# EUROPÄISCHE PATENTSCHRIFT

(11) **EP 0 744 906 B1**
(45) Veröffentlichungstag und Bekanntmachung des Hinweises auf die Patenterteilung: **20.09.2000**
(21) Anmeldenummer: 95909670.2
(22) Anmeldetag: 03.02.1995
(51) Int. Cl.: A43B 5/04, A61F 5/01

(54) **SCHUH, INSBESONDERE SPORTSCHUH ODER ORTHOPÄDISCHER STRUMPF, MIT SPRUNGGELENKSTABILISIERUNG**
SHOE, IN PARTICULAR SPORT SHOE OR ORTHOPAEDIC STOCKING WITH ANKLE STABILISATION
CHAUSSURE, NOTAMMENT CHAUSSURE DE SPORT OU BAS ORTHOPEDIQUE A STABILISATION DE LA CHEVILLE

(30) Priorität: 16.02.1994 DE 4404911
(43) Veröffentlichungstag der Anmeldung: 04.12.1996
(73) Patentinhaber: Ahlbäumer, Georg, CH-7500 St. Moritz (CH)
(72) Erfinder: Ahlbäumer, Georg, CH-7500 St. Moritz (CH)
(74) Vertreter: Prechtel, Jörg, Dipl.-Phys. Dr.
(86) Internationale Anmeldenummer: EP9500396
(87) Internationale Veröffentlichungsnummer: WO9522264

(56) Entgegenhaltungen:
- EP-A- 0 471 955
- AT-B- 385 636
- DE-U- 6 608 312
- DE-U- 8 814 157
- DE-U- 9 307 688
- US-A- 5 242 379

## Beschreibung

Die Erfindung betrifft einen Schuh, insbesondere Sportschuh, und einen orthopädischen Strumpf mit einer Sprunggelenkstabilisierung. Der Schuh bzw. der Strumpf umfaßt eine Verstärkung, die den Fuß unterhalb der Sprunggelenke teilweise oder vollständig umfaßt und seitlich des Fußes bis oberhalb des oberen Sprunggelenks reicht, wobei die Verstärkung ein den Fuß unterhalb der Sprunggelenke zumindest teilweise umgreifendes Bügelelement und ein den Unterschenkel oberhalb des oberen Sprunggelenks zumindest teilweise umgreifendes Manschettenelement aufweist. Der Schuh bzw. Strumpf umfaßt ferner Gelenkmittel, die das Manschettenelement mit dem Bügelelement verbinden zur Übertragung zumindest von Zugkräften zwischen Bügelelement und Manschettenelement. Die Gelenkmittel definieren eine effektive Bewegungsachse des Schuhs bzw. des Strumpfes.

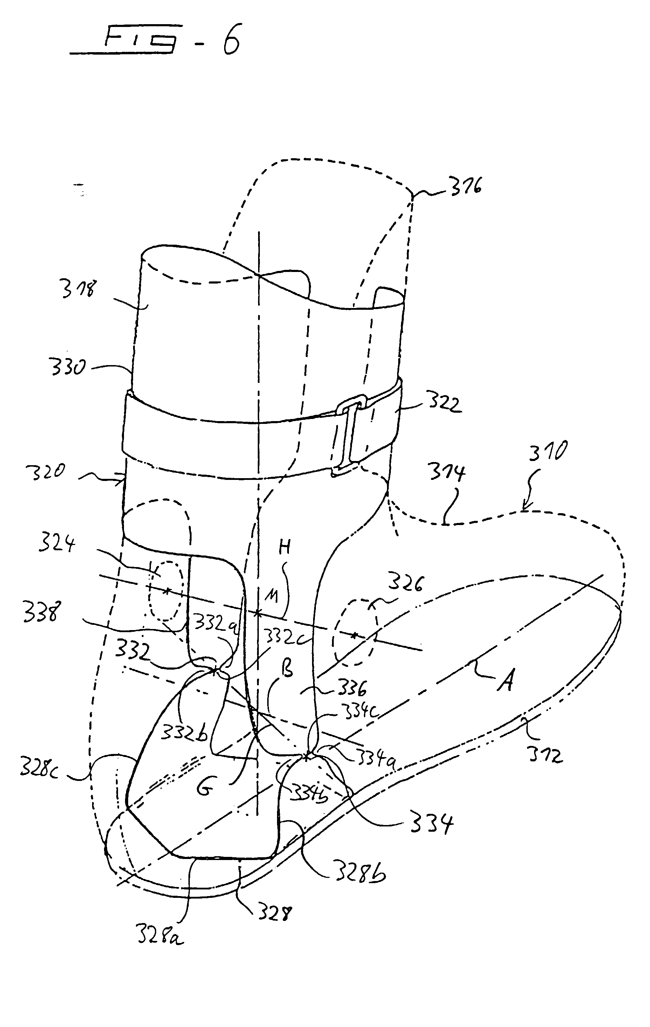

Ein derartiger Langlaufschuh ist aus der EP 0 416 437 B1 bekannt. Die Gelenkmittel bestehen aus einem fußaußenseitigen Gelenk und einem fußinnenseitigen Gelenk, die das Manschettenelement auf der Fußaußenseite und auf der Fußinnenseite mit dem Bügelelement verbinden. Um eine sichere Skiführung zu erreichen, ist das Manschettenelement über jeweils ein Gelenk mit seitlich hochgezogenen Lappen einer Hinterfußfersenschale verbunden, so daß dementsprechende Skiführungskräfte unter Entlastung der Sprunggelenke in den Unterschenkel übertragen werden können. Beide Gelenke haben gleiche Höhe γ, die etwa der Höhe der Knöchel entspricht, sowie gleichen Abstand x vom hinteren Schuhende (Fig. 6), so daß eine beide Gelenke verbindende Verbindungsgerade parallel zur zur Schuhlängsachse senkrechten Schuhquerachse verläuft. Beide Gelenke ermöglichen eine Extensionsbewegung und Flexionsbewegung, wie diese beim Langlauf erforderlich ist.

Aus dem deutschen Gebrauchsmuster DE 88 07 537 U1 ist ein Langlaufschuh bekannt, bei dem eine nur bis zum Knöchel reichende Manschette mit einer seitlichen Laufsohlenumwölbung an der Schuhaußenseite und an der Schuhinnenseite jeweils über dementsprechend tiefergelegene Gelenke verbunden ist, um eine für den klassischen Langlauf ausreichende Flexions- und Extensionsbeweglichkeit sicherzustellen und gleichzeitig dem Knöchel genügend Halt in seitlicher Richtung für die Skating-Technik zu bieten.

Aus der EP 0 356 400 A2 ist ein Skischuh der eingangs genannten Art bekannt, bei dem die Gelenkmittel aus einem fußaußenseitigen Gelenk und einem fußinnenseitigen Gelenk bestehen, die das Manschettenelement auf der Fußaußenseite und auf der Fußinnenseite mit dem Bügelelement verbinden. Um den Skischuh für ein optimales Führen der Skier, insbesondere ein optimales Gleiten beim Fahren in Vorlagestellung, auszulegen, ist bei dem bekannten Skischuh das fußaußenseitige Gelenk gegenüber dem fußinnenseitigen Gelenk in Schuh-Längsrichtung nach vorne versetzt angeordnet. Die Offenlegungsschrift geht dabei von der folgenden Ausnahme aus: Unter der Voraussetzung, daß der Fuß in seiner normalen, gerade nach vorne weisenden Lage unbeweglich auf seiner Auflage (dem Ski) belassen wird, kann die Relativbewegung zwischen dem Fuß und dem Unterschenkel durch eine Schwenkachse beschrieben werden, die schräg von lateral (fußaußenseitig) vorne nach medial (fußinnenseitig) hinten verläuft und mit einer zur Fußlängsachse orthogonalen Querachse einen Winkel von etwa 3° bis 4° einschließt. Hierdurch läßt sich eine beim Vorbeugen des Unterschenkels unter der genannten Voraussetzung nach den Angaben in der Offenlegungsschrift offenbar beobachtete Bewegungsrichtung des Knies schräg nach vorne und innen anschaulich erklären. Die Offenlegungsschrift möchte dieser Bewegung des Knies dadurch Rechnung tragen, daß die beiden Gelenke des Skischuhs auf der angenommenen Schwenkachse liegen. Das fußaußenseitige Gelenk ist demensprechend gegenüber dem fußinnenseitigen Gelenk nach vorne versetzt. Bezogen auf eine zu einer Schuh-Längsrichtung orthogonale Schuh-Querachse ergibt sich hieraus, daß eine die beiden Gelenke verbindende, mit der Schwenkachse zusammenfallende Gerade (Bewegungsachse) mit der Schuh-Querachse in Projektion auf eine Horizontalebene einen Winkel von etwa 3° bis 4° einschließt. Im Anspruch 2 der Offenlegungsschrift wird ein Winkelbereich von 1° bis 7°, vorzugsweise 3° bis 4°, beansprucht. In der Offenlegungsschrift wird als besonders wichtig herausgestellt, daß das innere Gelenk möglichst konzentrisch zum inneren Knöchel angeordnet ist. Dies kann bei dem bekannten Skischuh dadurch gewährleistet werden, daß die Gelenke im wesentlichen parallel zu einer zur angenommenen Schwenkachse lotrechten Ebene verschiebbar angeordnet sind.

Aus der US 5,242,379 ist eine ortopädische Fußgelenkstütze bekannt, umfassend eine Art Strumpf und zwei Stützvorrichtungen, die beidseitig des Fußes auf oder unter dem Strumpf zur Begrenzung der Bewegung des Fußes relativ zum Unterschenkel befestigt werden. Die jeweilige Stützvorrichtung umfaßt ein am Unterschenkel zu befestigendes oberes Teil und ein am Fuß zu befestigendes unteres Teil, die mittels eines Gelenks mit Spiel in Fußlängsrichtung und in Fußhöhenrichtung gegeneinander verschwenkbar sind.

Aufgabe der Erfindung ist es, einen Schuh (insbesondere einen Sportschuh) und einen orthopädischen Strumpf der eingangs genannten Art bereitzustellen, der Schutz vor Knöchel- und Bänderverletzungen, insbesondere des oberen Sprunggelenks, bietet. Durch die Erfindung sollen insbesondere Schäden an Bändern und Knochen im Sprunggelenkbereich vermieden werden, die durch spontan unkontrollierte Supinations- oder auch durch übermäßige Pronationsbewegungen auftreten können. Dabei soll die sonstige Fußbeweglichkeit (Unterschenkelabwinklung etc.) nicht übermäßig behindert werden.

Diese Aufgabe wird durch den Schuh mit den Merkmalen des Anspruchs 1 bzw. des Anspruchs 2 bzw. des Anspruchs 3 sowie durch den Strumpf mit den Merkmalen des Anspruchs 4 gelöst.

Bezugnehmend auf die Ansprüche 1, 2 und 4 wird durch die Anordnung der Gelenke relativ zueinander und relativ zur Mittelposition zwischen dem äußeren Knöchel und dem inneren Knöchel (Anspruch 1 und Anspruch 4) bzw. des fußaußenseitigen Gelenks relativ zur Mittelposition (Anspruch 2) erreicht, daß bei einer Inversionsbewegung des Fußes, insbesondere einer Supinationsbewegung des Fußes, Kräfte zunehmend über das Gelenk auf der Fußaußenseite von der Fußsohle zum Manschettenelement und dementsprechend zum Unterschenkel übertragen werden, mit einem entsprechenden Abbremsen und entsprechender Beschränkung der weiteren Supinations- oder Inversionsbewegung des Fußes. Auf diese Art und Weise werden Supinationsschäden, insbesondere Bänderrisse (fibulare Bänder, insbesondere das Ligamentum talofibulare anterius), sowie erst recht Knochenverletzungen (osterere Verletzungen, insbesondere Knöchelfrakturen) wirksam vermieden.

Bezugnehmend auf die Ansprüche 1, 3 und 4 wird durch die Anordnung der Gelenke relativ zueinander und relativ zur Mittelposition zwischen den Knöcheln (Anspruch 1 und Anspruch 4) bzw. des fußinnenseitigen Gelenks relativ zur Mittelposition (Anspruch 3) erreicht, daß bei einer Eversionsbewegung des Fußes, insbesondere einer Pronationsbewegung, Kräfte zunehmend über das fußinnenseitige Gelenk von der Fußsohle zu dem Manschettenelement und dementsprechend zum Unterschenkel übertragen werden, mit einer entsprechenden Abbremsung und einer entsprechenden Beschränkung der weiteren Eversions- oder Pronationsbewegung des Fußes. Pronations- oder Eversionsschäden, insbesondere Frakturen des äußeren Knöchels, können somit effektiv verhindert werden.

Die Abbremsung und Beschränkung der Supinations-/Inversions- bzw. Pronations/Eversionsbewegung des Fußes folgen aus der beanspruchten Lage der Gelenke bzw. des jeweiligen Gelenks des Schuhs bzw. Strumpfes und der sich hieraus ergebenden Orientierung der die Relativbeweglichkeit zwischen Manschettenelement und Bügelelement charakterisierenden Schwenk- oder Bewegungsachse des Schuhs bzw. Strumpfes. Zumindest bei größeren Inversions-bzw. Eversionsbewegungen des Fußes dreht der Fuß nämlich auch um die Henkesche Achse, nicht bzw. weniger jedoch um die durch die Gelenkmittel definierte (erfindungsgemäß um einen beträchtlichen Winkel anders orientierte) Bewegungsachse, die im Falle eines fußinnenseitigen und eines fußaußenseitigen Gelenks entsprechend Anspruch 1 oder 4 mit einer Verbindungsgerade zwischen den Gelenken des Schuhs bzw. Strumpfes zusammenfällt.

Die Bewegungs- oder Schwenkachse des Schuhs bzw. Strumpfes ist relativ zu einer orthopädischen Achse des oberen Sprunggelenks, die annähernd transversal durch den inneren und den äußeren Knöchel verläuft, derart geneigt, daß sie einen die Bewegung um die Henkesche Achse und infolge übermäßige Supinations- und Pronationsbewegungen hemmenden Winkel relativ zur Henkeschen Achse aufweist. Aufgrund der Grundelastizität des Schuhs bzw. des Strumpfes ist anfänglich trotz der unterschiedlichen Orientierung der tatsächlichen Bewegungsachsen von Fuß einerseits und Schuh bzw. Strumpf andererseits eine Inversions- bzw. Eversionsbewegung (Supinations- bzw. Pronationsbewegung) des Fußes möglich. Zunehmend wird diese Bewegung jedoch durch den Schuh bzw. Strumpf gehemmt. Erfindungsgemäß ist also die volle Beweglichkeit des Fußes innerhalb des Schuhs bzw. Strumpfes gemäß der Erfindung nicht beabsichtigt, um Verletzungen des Fußes, insbesondere der Knöchel und Bänder, so weit wie möglich auszuschließen.

Kern der Erfindung ist also die erfindungsgemäße Orientierung der Bewegungsachse (ggf. Verbindungsgerade zwischen dem fußaußenseitigen Gelenk und dem fußinnenseitigen Gelenk) in bezug auf die Querachse und damit zur Henkeschen Achse, die die Bewegung der Fußwurzel unter dem oberen Sprunggelenk charakterisiert. Die Henkesche Achse verläuft schräg von lateral hinten unten nach medial vorne oben und bildet mit der schon erwähnten annähernd transversal verlaufenden Achse des oberen Sprunggelenks eine Art heterogenes Kardangelenk.

Der Schutz des ligamentum talofibulare anterius bei Inversionsbewegung ist deshalb erfindungsgemäß besonders wirksam, da sich dieses Band in Schuh-Längsrichtung vor dem äußeren Knöchel befindet, d. h. etwa dort, wo sich erfindungsgemäß auch das äußere Gelenk befindet (vgl. Anspruch 1, 2 und 4), welches entsprechende Zugkräfte unmittelbar aufnehmen kann zur Entlastung des ligamentum talofibulare anterius. Wie schon erwähnt, ist die volle Beweglichkeit des Fußes innerhalb des erfindungsgemäßen Schuhs bzw. Strumpfs erfindungsgemäß bewußt nicht angestrebt, um Fußverletzungen so weit wie möglich auszuschließen. Es hat sich gezeigt, daß dennoch eine ausreichende Grundbeweglichkeit gegeben ist, da der Schuh bzw. Strumpf eine gewisse Nachgiebigkeit und Elastizität besitzt. Gleichwohl muß sowohl das Bügelelement als auch das Manschettenelement ausreichend zugsteif ausgebildet sein, um eine effektive Begrenzung der Inversions- bzw. Eversionsbewegung des Fußes zu erhalten. Die Entlastungskräfte werden unter Umgehung des oberen und unteren Sprunggelenks vom Unterschenkel über das Manschettenelement, das Gelenk und das Bügelelement auf die Fußunterseite übertragen, wobei letzterer Kraftübergang deshalb problemlos ist, weil das Körpergewicht auf dem den Fuß zumindest teilweise umgreifenden und ggf. einstückig mit einer Sohle des Fußes ausgebildeten Bügelelement lastet.

Ein momentanes Ausweichen der Fußsohle ist ausgeschlossen, wenn gemäß einer besonders bevorzugten Ausführungsform (vgl. die Ansprüche 1 und 4) das Bügelelement den Fuß vollständig umgreift und über das fußaußenseitige Gelenk und das fußinnenseitige Gelenk mit dem an dem Unterschenkel angreifenden Manschettenelement verbunden ist. Diese Anordnung hat den großen Vorteil, daß Schutz sowohl vor Pronationsschäden als auch vor Supinationsschäden geboten wird. Es hat sich überdies gezeigt, daß die erfindungsgemäße Schrägstellung der Verbindungsgeraden zwischen den beiden Gelenken nach außen vorne bzw. nach innen hinten die natürliche Abrollbewegung des Fußes erleichtert.

Die Erfindung wird bevorzugt bei zweiteiligen Schuhen am Innenschuh vorgesehen, um die Verstärkung möglichst nahe an den Fuß zu bringen und gesonderte Fußbewegungen innerhalb des Schuhs auszuschließen. Beispiele für zweiteilige bzw. zweischalige Schuhe mit der erfindungsgemäßen Verstärkung am Innenschuh sind Snowboardschuhe, Bergschuhe, Skischuhe usw.

Man kann die erfindungsgemäße Verstärkung jedoch auch an einteiligen Schuhen, wie Langlaufschuhen, Trekkingschuhen, Paraglidingschuhen, Waldlaufschuhen, orthopädischen Schuhen und Basketballschuhen, einsetzen.

Schließlich ist unter Umständen der Einsatz der erfindungsgemäßen Verstärkung auch am Außenschuh zweiteiliger bzw. zweischaliger Schuhe denkbar, wobei vor allem an enganliegende Außenschuhe gedacht ist. Selbst bei zweischaligen Schuhen mit harter äußerer Schale (Skischuh), bei denen deshalb Supinationsprobleme nicht auftreten, kann die erfindungsgemäße Schrägstellung der Verbindungsgeraden von Vorteil sein, da diese die natürliche Abrollbewegung des Fußes erleichtert.

Um Kollisionen der Knöchel mit den Gelenken des Schuhs auszuschließen und auch die Beweglichkeit des Schuhs bei normalen Bewegungsmustern zu verbessern, wird vorgeschlagen, daß das fußaußenseitige Gelenk gegenüber dem äußeren Knöchel des oberen Sprunggelenks nach unten versetzt angeordnet ist, bzw. daß das innenseitige Gelenk gegenüber dem inneren Knöchel des oberen Sprunggelenks nach unten versetzt angeordnet ist.

Eine Gelenkverletzungen zuverlässig verringernde Begrenzung der Inversions- bzw. Eversionsbewegung bei gleichzeitig ausreichender Grundbeweglichkeit erhält man bevorzugt dadurch, daß der die Orientierung der Bewegungsachse relativ zur Querachse kennzeichnende Winkel α 10° bis 30°, vorzugsweise etwa 20°, beträgt.

In Weiterbildung der Erfindung wird für den Fall, daß ein fußinnenseitiges und ein fußaußenseitiges Gelenk vorgesehen ist, vorgeschlagen, das das fußaußenseitige Gelenk höher als das fußinnenseitige Gelenk angeordnet ist und die Projektion der Verbindungsgeraden bzw. Bewegungsachse zwischen den beiden Gelenken auf eine zur Schuh-Längsrichtung senkrechte, die Schuh-Querachse enthaltende Vertikalebene und die Schuh-Querachse einen Winkel β von 5° bis 15°, vorzugsweise von etwa 10°, einschließen.

Es hat sich herausgestellt, daß eine derartige Neigung der Verbindungsgeraden zu einem Schuh bzw. Strumpf mit hohem Laufkomfort führt, wobei dennoch gegen Gelenkschäden Schutz geboten wird.

Eine Kollision der Knöchel mit Teilen der fußaußenseitigen bzw. fußinnenseitigen Gelenke bzw. mit den oberen Enden des Bügelelements wird dadurch ausgeschlossen, daß das fußinnenseitige Gelenk und/oder das fußaußenseitige Gelenk etwa auf halber Höhe zwischen der Position des äußeren Knöchels bzw. der Position des inneren Knöchels des oberen Sprunggelenks und der Oberseite der Innensohle angeordnet sind.

Es sind vielfältige Gestaltungen von Bügelelement und Manschettenelement denkbar. Bevorzugt ist vorgesehen, daß das Manschettenelement eine den Unterschenkel umgreifende, vorzugsweise durch ein Verschlußelement schließbare Manschette sowie ein zugsteifes Verbindungsteil zwischen Manschette und dem jeweiligen Gelenk, vorzugsweise in Form einer Zunge, aufweist. Die Zunge dient so der Übertragung von das Fußgelenk entlastenden Zugkräften.

Besonders bevorzugt ist vorgesehen, daß die Zunge und das Bügelelement drucksteif ausgebildet sind. Auf diese Weise können die das Fußgelenk entlastenden Kräfte als Zugkräfte und Druckkräfte an der Fußaußenseite und Fußinnenseite zwischen Fußsohle und Unterschenkel übertragen werden.

Besonders bevorzugt ist hierbei vorgesehen, daß die aus Leder oder dergleichen flexiblem Material gebildete Zunge durch eine Kunststoffplatte verstärkt ist. Das Manschettenelement kann so insgesamt aus körperfreundlichem, gut flexiblem Material, insbesondere Leder, gebildet sein mit lokaler Verstärkung der Zunge über die Kunststoffplatte, um die gewünschte Drucksteifigkeit zu erhalten.

Das Bügelelement kann als beide Gelenke verbindender ggf. gesonderter Bügel ausgebildet sein. Es ist jedoch auch möglich, das Bügelelement mit einer Sohle zu integrieren, insbesondere in denjenigen Fällen, in denen die Gelenke am Außenschuh vorgesehen sind. Bei einteiligem Schuh oder Gelenk an der Außenschale eines zweiteiligen Schuhs ist bevorzugt vorgesehen, daß zur Bildung des Bügelelements die von vornherein vorgesehene, im allgemeinen nach hinten geschlossene Hinterfuß-Fersenschale seitlich bis zu den Gelenken hochgezogen ist. Dieser gibt ausreichende Stabilität bei niedrigen Herstellungskosten.

Zusätzlich oder auch unabhängig von den vorstehend beschriebenen Maßnahmen zum Schutz des Schuhträgers vor Knöchelverletzungen kann vorgesehen sein, daß wenigstens eines der beiden Gelenke mit im wesentlichen vertikalem Gelenkspiel ausgebildet ist.

Größere Supinations- bzw. Pronationsbewegungen sind anfänglich ungehindert möglich, jedoch nur bis zur Erschöpfung des Gelenkspiels. Nach dem Anschlag werden die Kräfte über das jeweilige Gelenk von der Fußsohle zum Unterschenkel geleitet mit entsprechender Entlastung der Fußgelenke und Bänder.

Es sind vielfältige Gestaltungen des Gelenks bzw. der Gelenke denkbar. So kann das Bügelelement mit dem Manschettenelement zur Gelenkbildung über ein Gelenkbolzen-Element verbunden sein, welches in eine Ausnehmung, vorzugsweise ein Loch oder gegebenenfalls ein Langloch für vertikales Gelenkspiel, im Bügelelement und/oder im Manschettenelement eingreift. Es kann aber auch wenigstens ein Gelenk durch eine Materialabschwächung oder dergleichen gebildet sein. Eine weitere Möglichkeit besteht darin, daß wenigstens ein Gelenk durch einen Faltenbalgabschnitt oder dergleichen gebildet ist.

Insbesondere die Ausbildung des bzw. der Gelenke durch eine jeweilige Materialabschwächung oder dergleichen, beispielsweise in Form eines Filmgelenks, oder durch einen jeweiligen Faltenbalgabschnitt oder dergleichen macht es möglich, daß das Manschettenelement und das Bügelelement einstückig ausgebildet sind. Die Herstellungskosten des erfindungsgemäßen Schuhes werden hierdurch gesenkt. Das jeweilige Gelenk behält seine volle Funktionsfähigkeit auch bei Verschmutzung bei.

Ist wenigstens ein Gelenk durch einen Faltenbalgabschnitt oder dergleichen gebildet, so ist es bevorzugt, daß die die Falten des Faltenbalgabschnitts verbindenden Teilabschnitte des Faltenbalgabschnitts im wesentlichen in Schuh-Längsrichtung verlaufen und sich nach hinten oder nach vorne verbreitern. Hierdurch wird eine ausreichende Lokalisierung des fußinnenseitigen bzw. des fußaußenseitigen Gelenks an der erfindungsgemäßen Stelle relativ zu dem äußeren bzw. dem inneren Knöchel des oberen Sprunggelenks erreicht, unter Gewährleistung einer ausreichenden Zugsteifigkeit der gelenkigen Verbindung zwischen Manschettenelement und Bügelelement, und gegebenenfalls nach einer definiert begrenzten anfänglichen Verlängerung des jeweiligen Faltenbalgabschnitts, also einem vertikalen Gelenkspiel.

Das Manschettenelement kann bezüglich des Bügelelements abnehmbar sein. In diesem Zusammenhang ist es besonders bevorzugt, daß wenigstens zwei unterschiedliche Manschettenelemente, insbesondere mit unterschiedlichen Abmessungen insbesondere in Vertikalrichtung vorgesehen sind, die wahlweise zur Anpassung des Schuhes an die Anatomie des Trägers und/oder zur Anpassung des Schuhes an den Verwendungszweck mit dem Bügelelement verbindbar sind. Durch diese Maßnahme braucht nur eine verringerte Anzahl von Schuhvarianten gefertigt werden, wodurch sich die Herstellungskosten reduzieren. Auch der Träger kann gegebenenfalls den Schuh an den Verwendungszweck anpassen, so daß der Schuh einen höheren Gebrauchswert für den Träger besitzt. Die genannte Abnehmbarkeit des Manschettenelements und das Vorsehen unterschiedlicher Manschettenelemente ist insbesondere dann zweckmäßig, wenn die erfindungsgemäße Verstärkung an der Außenschale eines zweischaligen Schuhes oder an einem einschaligen Schuh vorgesehen ist.

Das Manschettenelement kann bezüglich des Bügelelements höhenverstellbar sein. Durch diese Maßnahme ist ebenfalls eine Anpassung des Schuhes an die Anatomie des Trägers und/oder an den Verwendungszweck möglich; es ergeben sich die vorgenannten Vorteile.

Wie schon deutlich wurde, läßt sich die vorstehend beschriebene erfindungsgemäße Verstärkung zur Sprunggelenkstabilisierung mit Vorteil auch in Verbindung mit einem orthopädischen Strumpf oder dergleichen textilen Stützgeweben einsetzen (vgl. Anspruch 4). Der fürdie gewünschte Stabilisierungswirkung erforderliche Kraftschluß zwischen Fußunterseite und Bügel ist dann, wenn erforderlich, gewährleistet, nämlich zum Zeitpunkt des Auftretens, da dann der Fuß aufgrund des plötzlichen Abbremsens von Fuß und Körper mit momentan hoher Kraft auf den Bügel drückt und so einen momentanen Reibungskraftschluß herstellt.

Das Bügelelement kann folglich der Einfachheit halber von einem zugsteifen Band gebildet sein, ebenso wie das Manschettenelement von einem den Unterschenkel umgreifenden Verschlußband und einem fußinnenseitigen und einem fußaußenseitigen zugsteifen Verbindungsteil, vorzugsweise in Form einer Zunge, gebildet sein kann. Es kommt aber auch grundsätzlich eine Ausbildung des erfindungsgemäßen Strumpfes entsprechend den vorstehend genannten und in den Unteransprüchen angegebenen Aus- und Weiterbildungen eines erfindungsgemäßen Schuhs in Betracht.

Die Erfindung wird im folgenden an einem bevorzugten Ausführungsbeispiel anhand der Zeichnungen erläutert. Es zeigt:
- Figur 1: eine perspektivische Ansicht eines Innenschuhs (mit unterbrochener Umrißlinie) für einen jweiteiligen Snowboard-Schuh mit erfindungsgemäßer Verstärkung;
- Figur 2: eine mediale Ansicht des Innenschuhs gemäß Fig. 1 (Blickrichtung II in Figur 1);
- Figur 3: eine laterale Ansicht des Innenschuhs gemäß Figuren 1 und 2 (Blickrichtung III in Figur 1);
- Figur 4: eine perspektivische Ansicht eines orthopädischen Strumpfes mit erfindungsgemäßer Verstärkung;
- Figur 5: eine perspektivische Ansicht einer anderen Ausführungsform eines erfindungsgemäßen Schuhes entsprechend dem Innenschuh der Figur 1;
- Figur 6: eine perspektivische Ansicht einer weiteren Ausführungsform eines erfindungsgemäßen Schuhes entsprechend dem Innenschuh der Figur 1;
- Figur 7: eine laterale Ansicht einer weiteren Ausführungsform des erfindungsgemäßen Schuhes in einer Ansicht entsprechend Figur 3; und
- Figur 8: in den Teilfiguren a) und b) Schnitte durch den in Figur 7 gezeigten, das fußaußenseitige Gelenk bildenden Faltenbalgabschnitt nach den Linien VIIIa-VIIIa bzw. VIIIb-VIIIb.

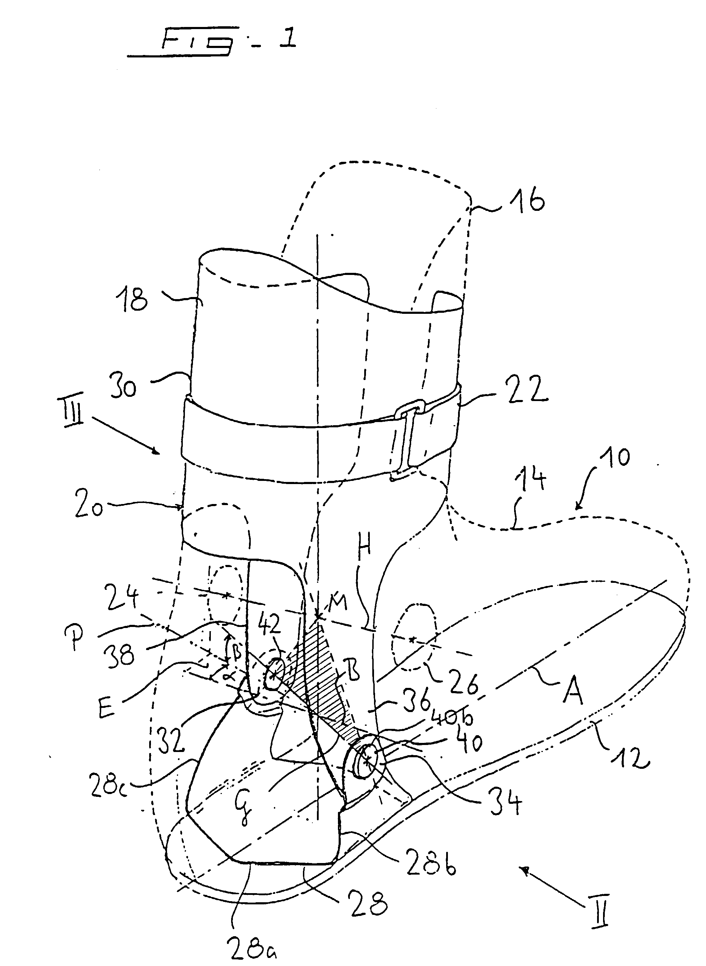

Der in den Figuren 1 bis 3 vereinfacht dargestellte Innenschuh 10 aus Sohle 12 und Schaft 14 einschließlich Zunge 16 ist mit einer Manschette 18 als Teil einer insgesamt mit 20 bezeichneten Verstärkung versehen. Die Manschette 18 kann mit einem Verschlußelement in Form eines Verschlußbandes 22 um den Unterschenkel des Schuhträgers oberhalb seines äußeren und inneren Knöchels (in den Figuren jeweils durch einen Kreis 24 bzw. 26 angedeutet) verschlossen werden. Es kann sich um ein Klettverschluß-Band handeln.

Die bereits erwähnte Verstärkung 20 wird von einem den Fuß unterhalb der beiden Sprunggelenke umgreifenden Bügelelement 28 sowie einem die Manschette 18 umfassenden Manschettenelement 30 gebildet mit Verbindung beider Elemente 28 und 30 über jeweils ein fußaußenseitiges Gelenk 32 und fußinnenseitiges Gelenk 34.

Im dargestellten Ausführungsbeispiel ist das Bügelelement 28 als gesondertes, im Querschnitt senkrecht zur Schuh-Längsrichtung A im wesentlichen U-förmiges Teil ausgebildet. Der Mittelschenkel 28a der U-Form kann, wie dargestellt, an der Oberseite der beispielsweise aus festem Kunststoff bestehenden Sohle 12 des Innenschuhs 10 angeordnet sein, d. h. unmittelbar unterhalb der nicht dargestellten Brandsohle oder Einlegesohle, so daß quasi unmittelbarer Kontakt mit der Fußsohle des Trägers besteht. Die beiden Seitenschenkel 28b und 28c der U-Form tragen an ihren freien oberen Enden jeweils das Gelenk 34 bzw. 32. Sie überlappen hierzu mit Enden von beidseits des Innenschuhs 10 von der Manschette 18 nach unten abstehenden Zungen 36 und 38 als Teil des Manschettenelements 20. Zusammengehalten werden die Zungenenden und die Schenkelenden jeweils durch ein Gelenkbolzenelement, vorzugsweise in Form eines Nietelements 40 bzw. 42.

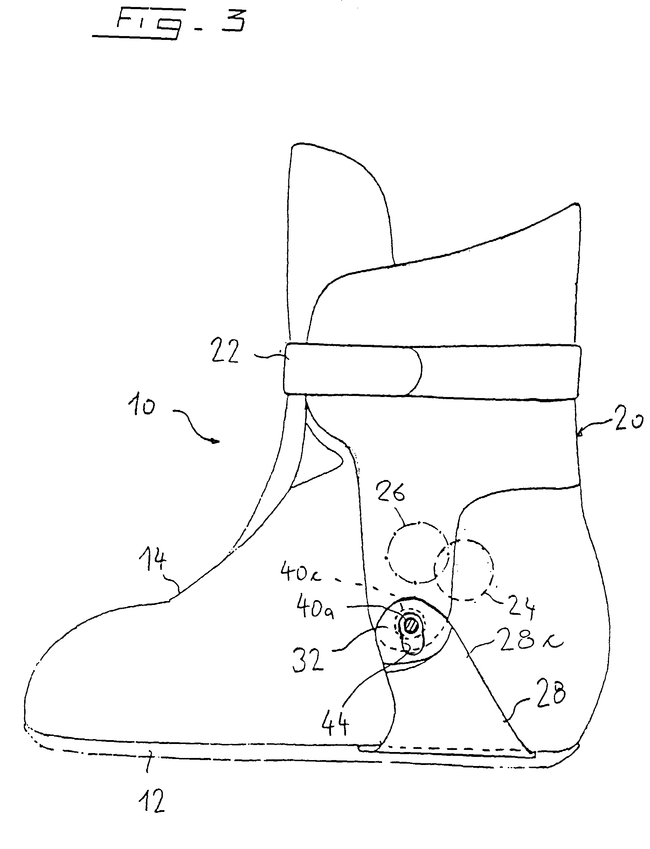

Um eine anschlagsbegrenzte Relativbeweglichkeit zwischen Bügelelement 28 und Manschettenelement 40 zu erhalten, kann ein entsprechend vertikales Gelenkspiel in einem oder besser in beiden Gelenken 32 und 34 vorgesehen sein. Dies kann gemäß Figur 3 beispielsweise dadurch realisiert werden, daß im oberen Ende des jeweiligen Schenkels 28b bzw. 28c ein im wesentlichen vertikal orientiertes Langloch 44 ausgebildet ist, welches vom Schaft 40a des Nietelements 40 durchsetzt ist (in Fig. 3 ist der außenseitige Kopf 40b des Nietelements 40 der Übersichtlichkeit halber weggelassen und nur der innenseitige Kopf 40c mit unterbrochener Linie angedeutet). Aufgrund dieses Spiels wird eine Verkippungsbewegung des Manschettenelements 30 gegenüber dem Bügelelement 28 anschlagsbegrenzt, so daß dementsprechend auch eine Supinationsbewegung bzw. Pronationsbewegung des Fußes gegenüber dem Unterschenkel in vorgegebenen Maße anschlagsbegrenzt ist, um Knöchelverletzungen zu vermeiden.

Unabhängig hiervon, jedoch am besten zusätzlich, wird ein Verletzungsschutz dadurch erreicht, daß die beiden Gelenke 32 und 34 keinesfalls in Richtung der Schuh-Querachse B (senkrecht zur Längsrichtung A und in horizontaler Ebene E liegend) miteinander fluchten, sondern in Längsrichtung versetzt sind. Das fußaußenseitige (= laterale) Gelenk 32 ist in Längsrichtung A nach vorne versetzt und das fußinnenseitige (= mediale) Gelenk 34 dementsprechend nach hinten versetzt.

Gemäß Figur 1 kann das Ausmaß der Versetzung durch einen Winkel α beschrieben werden, der von einer Projektion P der Verbindungsgeraden G zwischen beiden Gelenken 32 und 34 auf die die Querachse B enthaltende Horizontalebene E und der Querachse B eingeschlossen ist.

Bezogen auf den Fuß des Schuhträgers kann die Versetzung der Gelenke 32 und 34 auch derart beschrieben werden, daß das äußere Gelenk 32 gegenüber dem Mittelpunkt M der Verbindungsstrecke H zwischen beiden Knöcheln (Kreise 24 und 26) nach vorne versetzt ist und dementsprechend das innere Gelenk 34 nach hinten.

Der Winkel α beträgt 10° bis 30°, vorzugsweise etwa 20°.

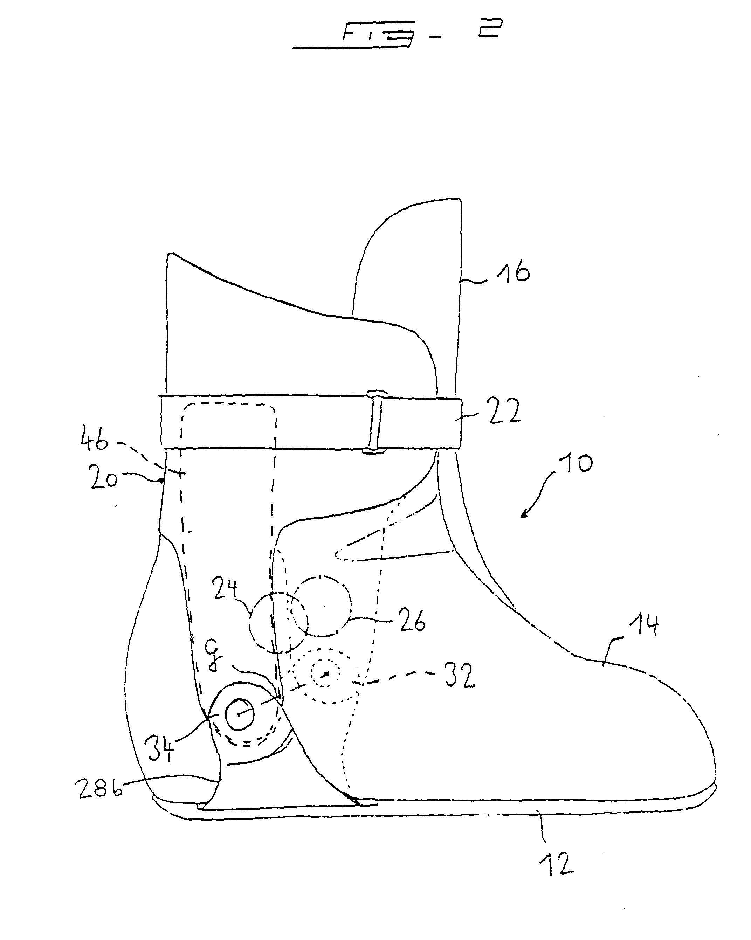

Desweiteren sind die Gelenke 32 und 34 gegenüber den Knöcheln (Kreise 24 und 26) nach unten versetzt, etwa bis auf halbe Höhe. Das fußinnenseitige Gelenk 34 liegt dabei tiefer als das fußaußenseitige Gelenk 32, wie insbesondere Figur 2 zeigt. Der Neigungswinkel der Verbindungsgeraden G zur Horizontalebene E ist in Figur 1 mit β bezeichnet. Er beträgt 5° bis 15°, vorzugsweise etwa 10°.

Aufgrund der Raumorientierung der Gelenke 32 und 34 ergibt sich ein wirksamer Schutz des Schuhträgers vor Pronationsschäden und Supinationsschäden des Fußgelenks. Gleichzeitig ist eine ausreichende Beweglichkeit gewährleistet.

Die Vor- bzw. Rückverlagerung der Gelenke 32 bzw. 34 entsprechen dem Winkel α führt zu einer zunehmenden Zugbelastung von Zunge 38, Gelenk 32 und Seitenschenkel 28c bei zunehmender Supinationsbewegung oder Inversionsbewegung. Dies liegt daran, daß im Laufe der Supinationsbewegung bzw. Inversionsbewegung der Fuß zunehmend um die Henkesche Achse verschwenkt. Diese Achse ist entgegengesetzt zur Verbindungsgeraden G zwischen beiden Gelenken 32 und 34 orientiert, also von hinten lateral, nach vorne medial. Dementsprechend wird das Gelenk 32 zunehmend auf Zug belastet und das Gelenk 34 zunehmend auf Druck unddie weitere Supinationsbewegung bzw. Inversionsbewegung gehemmt.

Der an sich besonders gefährdete Ligamentum talofibulare anterius verläuft vor dem äußeren Knöchel (Kreis 24) ebenso wie Zunge 38, Gelenk 32 und Seitenschenkel 28c, so daß dieses Gelenkband unmittelbar durch die dortige erfindungsgemäße Verstärkung vor übermäßiger Zugbeanspruchung geschützt ist.

Bei Pronationsbewegung bzw. Eversionsbewegung wird das Gelenk 32 dagegen auf Druck und das Gelenk 34 auf Zug belastet mit entsprechender Beschränkung der Fußgelenkbelastung. Der Verletzungsschutz ist bereits dann wirksam, wenn die Verstärkung lediglich zur Übertragung von Zugkräften zwischen Fußsohle und Unterschenkel über die beiden Gelenke ausgebildet ist.

Eine weitere Verbesserung besteht jedoch darin, daß durch die Verstärkung auf der entsprechenden Seite auch Druckkräfte aufgenommen und weitergeleitet werden, wozu das Manschettenelement 30 dementsprechend verstärkt ist. Möchte man aus tragephysiologischen Gründen eine entsprechend drucksteife Manschettenschale vermeiden, so kann man, wie in Figur 2 angedeutet, ein gesondertes Verstärkungselement in Form einer Kunststoffplatte 46 einsetzen, die beispielsweise mit der entsprechenden Zunge 36 bzw. 38 als Unterlage vernäht ist oder in eine entsprechend dimensionierte Tasche des Manschettenelements eingesetzt ist. Die Kunststoffplatte 46 reicht vom jeweiligen Gelenk 34 bis zum Verschlußband 22, um die Druckkräfte weiterleiten zu können. Da auch das Gelenk 34 bzw. 32 selbst (ggf. bis auf das eingangs besprochene Gelenkspiel) sowie die sich anschließenden Seitenschenkel 28b bzw. 28c drucksteif ausgebildet sind, können die Druckkräfte zwischen Fußsohle und Oberschenkel unter Umgehung der Fußknöchel über die Verstärkung weitergeleitet werden.

Der vorstehend beschriebene Innenschuh zeichnet sich durch hohen Verletzungsschutz bei ausreichender Beweglichkeit aus. Die erfindungsgemäße Verstärkung ist ohne größeren Material- und Herstellungsaufwand zu realisieren.

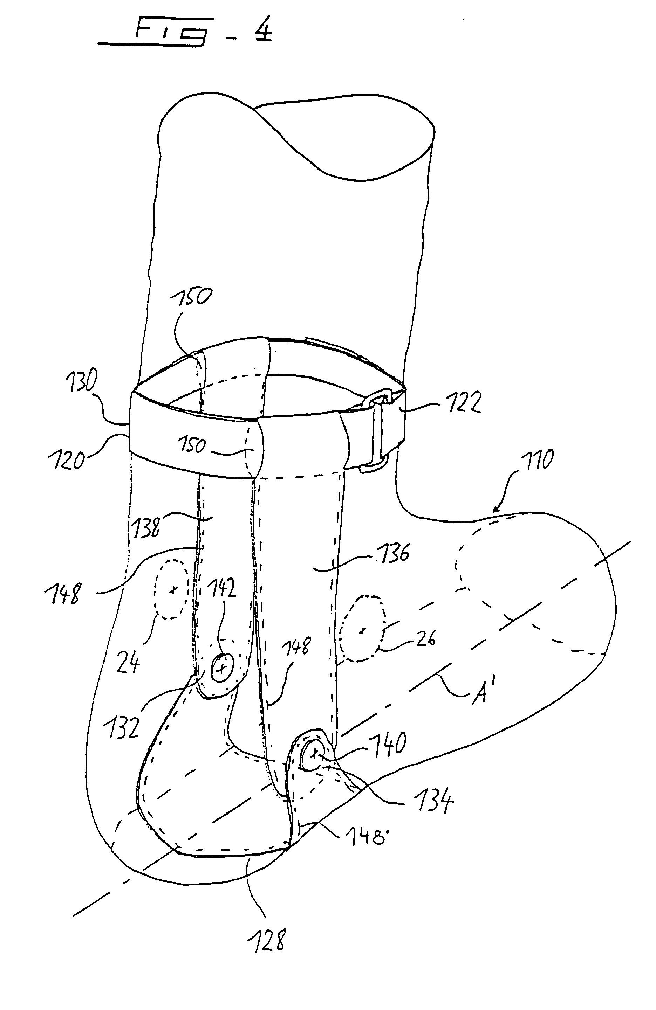

Die Verwendung der erfindungsgemäßen Verstärkung bei einem orthopädischen Strumpf ist in Figur 4 gezeigt. Der orthopädische Strumpf 110, der bei diesem Beispiel eine Öffnung für die Zehen aufweist, ist mit einer Verstärkung 120 versehen. Die Verstärkung 120 besteht aus einem Manschettenelement 130, dem fußinnenseitigen Gelenk 134, dem fußaussenseitigen Gelenk 132 und dem Bügelelement 128. Das Manschettenelement 130 wird von einem zugsteifen Verschlußband 122 und zugsteifen Zungen 136 und 138 gebildet. Die Zungen 136 und 138, die von zugsteifen Bändern gebildet sein können, weisen an ihrem oberen Ende Schlaufen 150 auf, durch welche das Verschlußband 122, das ein Klettverschluß-band sein kann, hindurchgeführt ist. Die Zungen 136 und 138 sind mit dem orthopädischen Strumpf 110 vernäht, die entsprechenden Nähte 148 sind gestrichelt angedeutet, wobei die Zungen 136 und 138 zweckmäßigerweise an die Außenseite des eigentlichen Strumpfes angenäht sind. Das Verschlußband 122 umgreift den Unter-schenkel des Strumpfträgers oberhalb seines äußeren und inneren Knöchels (durch einen Kreis 24 bzw. 26 angedeu-tet). Das Bügelelement 128, das den Fuß unterhalb der beiden Sprunggelenke umgreift, kann ebenfalls als ein zugsteifes an der Außenseite des Strumpfes 110 mit diesem vernähtes (Nähte 148') Band ausgebildet sein. Es bildet an seinen freien oberen Enden zusammen mit dem unteren Ende der jeweiligen Zunge 136 bzw. 138 die Gelenke 132 und 134, indem das Bügelelement 128 und die unteren Enden der Zungen 136 und 138 überlappen. Die oberen Enden des Bügelelements 128 und die unteren Enden der Zungen 136 und 138 werden vorzugsweise durch ein Nietelement 140 bzw. 142 gelenkig zusammengehalten.

Genau wie bei der im Zusammenhang mit den Figuren 1 bis 3 beschriebenen erfindungsgemäßen Verstärkung eines Innenschuhs, sind die beiden Gelenke 132 und 134 in der Längsrichtung des Strumpfes gegeneinander versetzt. Das fußaußenseitige (= laterale) Gelenk 132 ist in Längsrichtung A' nach vorne versetzt und das fußinnenseitige (= mediale) Gelenk 134 dementsprechend nach hinten versetzt. Bezogen auf den Fuß des Strumpfträgers ist das äußere Gelenk 132 gegenüber dem Mittelpunkt der Verbindungsstrecke zwischen den beiden Knöcheln (Kreise 24 und 26) nach vorne versetzt und dementsprechend das innere Gelenk 134 gegenüber dem Mittelpunkt der Verbindungsstrecke nach hinten.

Des weiteren sind die Gelenke 132 und 134 gegenüber den Knöcheln (Kreise 24 und 26) nach unten versetzt, etwa bis auf halbe Höhe. Das fußinnenseitige Gelenk 134 liegt dabei tiefer als das fußaußenseitige Gelenk 132.

Die Versetzung der Gelenke 132 und 134 gegenüber den Knöcheln (Kreise 24 und 26) bzw. gegenüber dem jeweils anderen Gelenk läßt sich wie bei dem Innenschuh der Figuren 1 bis 3 durch einen Winkel α und einen Winkel β (die Winkel sind in der Figur 4 nicht gezeigt) beschreiben. Der die Versetzung in Längsrichtung A' beschreibende Winkel α beträgt 10° bis 30°, vorzugsweise etwa 20°. Der Winkel β, der die gegenseitige Höhenversetzung der Gelenke 132 und 134 beschreibt, beträgt 5° bis 15°, vorzugsweise etwa 10°.

Aufgrund der Raumorientierung der Gelenke 132 und 134 ergibt sich ein wirksamer Schutz des Strumpfträgers vor Pronationsschäden und Supinationsschäden des Fußgelenks, da für diesen Schutz die Übertragung von Zugkräften zwischen Fußsohle und Unterschenkel ausreicht. Gleichzeitig ist eine ausreichende Beweglichkeit gewährleistet.

Der vorstehend beschriebene orthopädische Strumpf zeichnet sich durch hohen Verletzungsschutz bzw. in einer großen therapeutischen Wirksamkeit in der Stabilisierung der Sprunggelenke bei ausreichender Beweglichkeit aus. Die erfindungsgemäße Verstärkung ist ohne größeren Materialund Herstellungsaufwand zu realisieren.

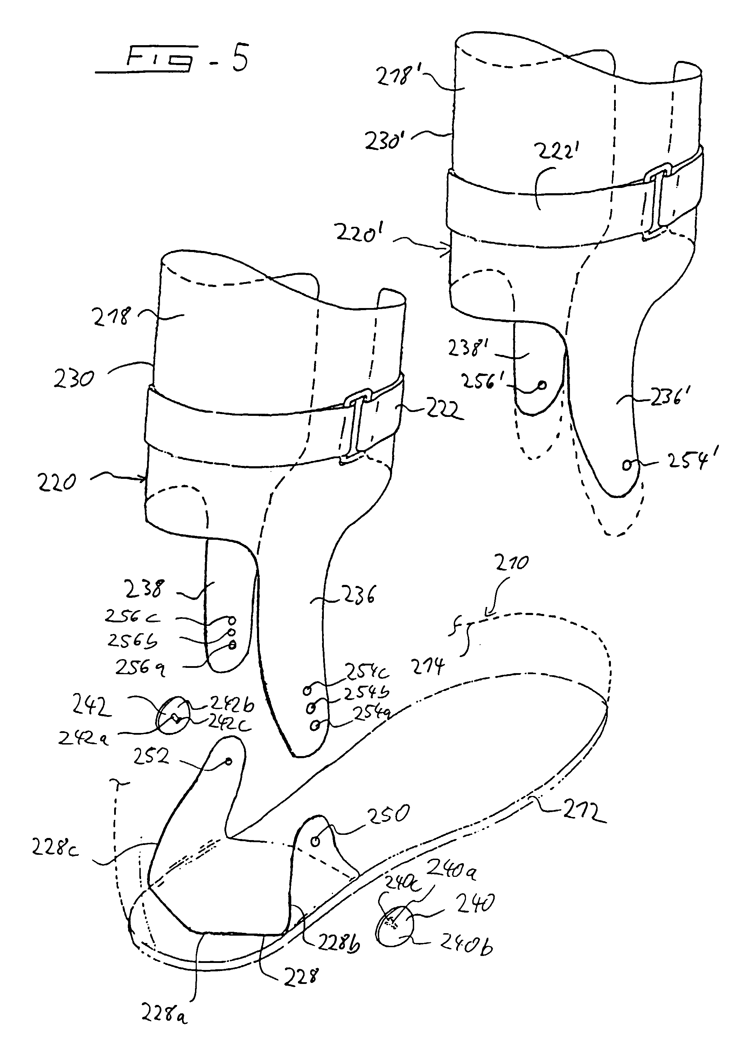

Im folgenden werden anhand der Figuren 5 bis 8 weitere Ausführungsformen eines erfindungsgemäßen Schuhes erläutert, der im grundsätzlichen Aufbau weitgehend dem in den Figuren 1 bis 3 gezeigten erfindungsgemäßen Innenschuh entspricht. Bauteile, die in ihrer Funktion denjenigen der ersten Ausführungsform gemäß den Figuren 1 bis 3 entsprechen, sind mit denselben Bezugsziffern versehen, jedoch jeweils von Ausführungsform zur Ausführungsform vermehrt um die Zahl 100. Es wird im folgenden jeweils nur auf die Unterschiede zwischen den Ausführungsformen eingegangen und im übrigen ausdrücklich auf die vorangehende Beschreibung der übrigen Ausführungsformen Bezug genommen.

Figur 5 zeigt einen erfindungsgemäßen Schuh, bei dem das Manschettenelement 320 samt der Manschette 218 und den Zungen 236 und 238 von dem Bügelelement 228 abnehmbar ist. Hierzu sind das innere Nietelement 240 und das äußere Nietelement 242 aus den zur Gelenkbildung dienenden Löchern 250 bzw. 252 im jeweiligen Seitenschenkel 228b bzw. 228c des Bügelelement 228 sowie aus dem jeweiligen zur Gelenkbildung dienenden Loch in der inneren Zunge 236 bzw. in der äußeren Zunge 238 entfernbar und wieder einsetzbar. Die Zungen 236 und 238 weisen jeweils drei übereinander angeordnete Löcher 254a, b, c bzw. 256a, b, c auf, wodurch eine einfache Höhenverstellbarkeit des Manschettenelements 230 bezüglich des Bügelelements 228 gegeben ist.

Die Nietelemente 240 und 242 weisen jeweils einen Schaft 240a bzw. 242a, einen außenseitigen Kopf 240b bzw. 242b bzw. einen innenseitigen Kopf 240c bzw. 242c auf. Der innenseitige Kopf 240c bzw. 242c besitzt einen wesentlich geringeren Durchmesser als der jeweilige außenseitige Kopf und ist derart bemessen, daß er unter elastischer Kompression des innenseitigen Kopfes bzw. unter elastischer Erweiterung des betreffenden Loches durch das jeweilige Loch im Bügelelement 228 bzw. in das jeweilige Loch in der inneren bzw. äußeren Zunge 236 bzw. 238 hindurchführbar ist zur gelenkigen Verbindung des Manschettenelements 230 mit dem Bügelelement 228 bzw. zur Lösung dieser gelenkigen Verbindung. Eine ungewollte Lösung der Verbindung zwischen dem Bügelelement und dem Manschettenelement ist dadurch ausgeschlossen, daß der Nietkopf 240c bzw. 242c schuhinnenseitig einrastet und zum Lösen der Verbindung folglich eine Kraft aufgebracht werden muß.

Zur optimalen Anpassung des Schuhes an die Anatomie des Trägers bzw. an den Verwendungszweck des Schuhes können auch mehrere Schuhoberteile einschließlich Manschettenelement vorgesehen sein, die insbesondere unterschiedliche Abmessungen, z. B. unterschiedliche Längen der Zungen für eine Höhenanpassung aufweisen. In Figur 5 ist ein derartiges Manschettenelement 230' mit kürzeren Zungen 236' bzw. 238' als die Zungen 236 bzw. 238 des Manschettenelements 230 gezeigt, das alternativ mit dem Bügelelement 238 mittels der Nietelemente 240 bzw. 242 verbindbar ist.

Eine weitere Ausführungsform des erfindungsgemäßen Schuhes ist in Figur 6 gezeigt. Hier ist die Verstärkung 320 aus Manschettenelement 330 und Bügelelement 328 einstückig ausgebildet. Das fußinnenseitige Gelenk 334 und das fußaußenseitige Gelenk 332 werden durch eine jeweilige Materialabschwächung gebildet. Die Seitenschenkel 328b und 328c des Bügelelement 328 sind demgemäß jeweils ein Teil der jeweiligen Zunge 336 bzw. 338. Zur Gelenkbildung ist die Zungenbreite der Zunge 336 bzw. 338 in Schuhlängsrichtung im Bereich des jeweiligen Gelenks deutlich reduziert unter Ausbildung einer jeweiligen vorderseitigen Einkerbung 334a bzw. 332a und einer jeweiligen hinterseitigen Einkerbung 334b bzw. 332b. Der zwischen dem jeweiligen oberen Zungenabschnitt der Zunge 336 bzw. 338 und dem jeweiligen Seitenschenkel 328b bzw. 338c übrig bleibende Steg 334c bzw. 332c ist biegbar und bildet folglich eine gelenkige Verbindung zwischen dem Manschettenelement 330 und dem Bügelelement 328. Der Steg 334c bzw. 332c kann durch ein geeignetes biegbares Verstärkungsmaterial, z. B. einem Draht oder dergleichen für eine besonders hohe Belastbarkeit verstärkt sein.

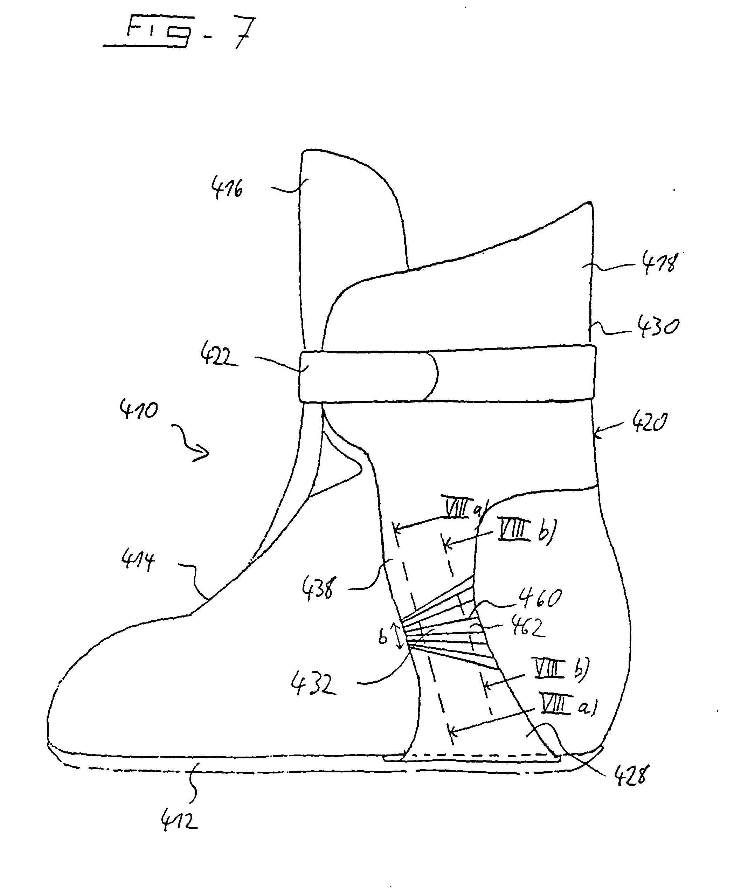

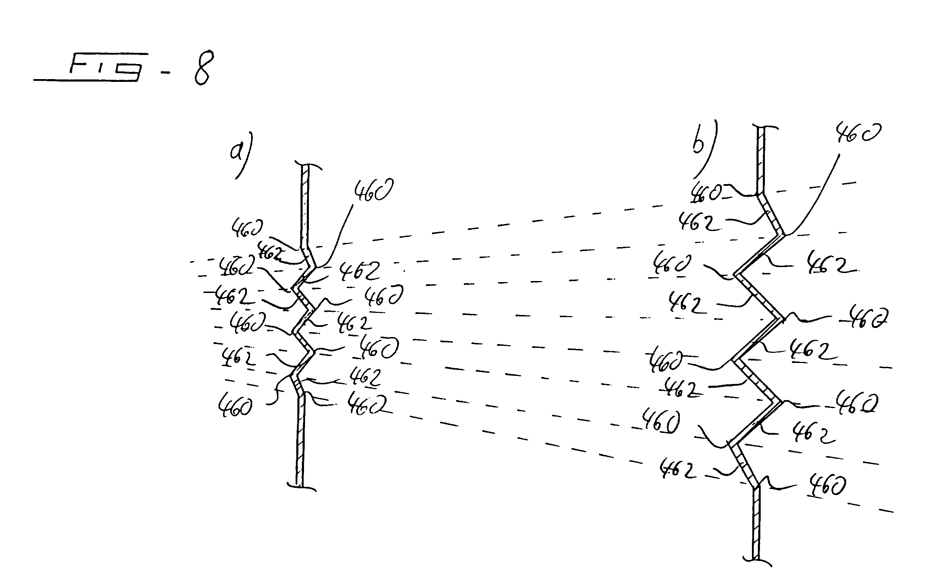

In den Figuren 7 und 8 ist eine weitere Ausführungsform eines erfindungsgemäßen Schuhes gezeigt. Hier ist das fußaußenseitige Gelenk 432 und das nicht gezeigte fußinnenseitige Gelenk durch einen jeweiligen, als Faltenbalgabschnitt bezeichneten ziehharmonikaartigen Abschnitt gebildet. Die Faltenbalgabschnitte können mit dem Bügelelement 428 und dem Manschettenelement 430 einteilig oder auch als gesonderte Teile ausgebildet sein.

Der insbesondere in den Figuren 8a und 8b erkennbare zickzackartige Verlauf der Faltenbalgabschnitte ermöglicht eine elastische Dehnbarkeit sowie eine elastische Zusammendrückbarkeit des Faltenbalgabschnitts in vertikaler Richtung. Eine Dreh- bzw. Schwenkbewegung des Manschettenelements 430 relativ zum Bügelelement 428 wird dadurch ermöglicht, daß beispielsweise die Faltenbalgabschnitte in einem vorderen (dem Zehenabschnitt näher liegenden) Bereich zusammengedrückt und in einem hinteren (dem Fersenabschnitt näher liegenden) Bereich gedehnt werden, und umgekehrt. Die Faltenbalgabschnitte üben also die erfindungswesentliche Gelenkfunktion aus.

Aufgrund der Dehnbarkeit der Faltenbalgabschnitte ist prinzipiell ein inhärentes Gelenkspiel in vertikaler Richtung gegeben.

Die die Falten 460 des Faltenbalgabschnitts verbindenden Teilabschnitte 462 des jeweiligen Faltenbalgabschnitts verlaufen im wesentlichen in Schuh-Längsrichtung und verbreitern sich nach hinten, um eine Lokalisierung des Gelenkpunkts im vorderen Bereich des jeweiligen Faltenbalgabschnitts zu erreichen. Aufgrund der genannten Ausbildung ist im hinteren Bereich des Faltenbalgabschnitts eine größere Dehnbarkeit bzw. Zusammendrückbarkeit als vorne gegeben. Der Abstand zwischen Manschettenelement und Bügelelement wird sich bei einer Drehbewegung im vorderen Bereich des jeweiligen Faltenbalgabschnitts deshalb weniger ändern als im hinteren Bereich des Faltenbalgabschnitts; der Gelenkpunkt ist folglich mehr im vorderen Bereich des Faltenbalgabschnitts lokalisiert.

Gegenüber einem Fall mit Faltenbalgabschnitten mit Teilabschnitten konstanter Breite ist - für eine vorgegebene Gesamt-Zugsteifigkeit des Faltenbalgabschnitts als Ganzes gegen eine Verlängerung - bei einer Ausbildung des Faltenbalgabschnitte entsprechend Figur 7 der Widerstand der Faltenbalgabschnitte gegenüber einer Drehbewegung bzw. Schwenkbewegung zwischen Manschettenelement und Bügelelement (bei gleicher Gesamt-Zugsteifigkeit) vermindert.

Das Gelenkspiel in vertikaler Richtung steht im Zusammenhang mit der vertikalen Abmessung b des Faltenbalgabschnitts bzw. mit der Breite der Teilabschitte 462 an der Vorderkante der erfindungsgemäßen Verstärkung. Im Grenzfall, daß b und damit die Breite der Teilabschnitte 462 an dieser Stelle gegen Null geht, die Falten 460 also in einem Punkt zusammenlaufen, ist kein vertikales Gelenkspiel mehr gegeben und der Gelenkpunkt fällt mit diesem Punkt, in dem die Falten 460 zusammenlaufen, zusammen. In diesem Fall ist der Gelenkpunkt des durch den Faltenbalgabschnitt gebildeten Gelenks also exakt lokalisiert.

Bei dem in Figur 7 gezeigten Ausführungsbeispiel ist eine derartige Lokalisierung des Gelenkpunktes nicht gegeben, der Gelenkpunkt ist aber auf eine relativ kleine Fläche lokalisiert. Dies ist aber unschädlich für die erfindungsgemäße Schutzfunktion, da auch die Fußbewegung rein physiologisch nicht durch eine feststehende Achse gekennzeichnet ist. Die Stellung der physiologischen Fußdrehachse ändert sich je nach Flexionsgrad und überstreicht dabei auf der Fußoberfläche eine kleine Fläche. Folglich reicht es aus, wenn die Gelenkpunkte der Faltenbalgabschnitte im wesentlichen entsprechend der genannten Fläche lokalisiert sind; die Gelenkachse des Schuhes kann sich also bei der Flexionsbewekung in einem gewissen Maße ändern, ohne die Schutzfunktion und den Komfort zu beeinträchtigen.

Zusammenfassend wird bei einem Schuh, insbesondere Sportschuh, mit einer den Fuß unterhalb der Sprunggelenke teilweise oder vollständig umfassenden, auf einer oder auf beiden Seiten des Fußes bis oberhalb des oberen Sprunggelenks reichenden Verstärkung, wobei die Verstärkung ein den Fuß unterhalb der Sprunggelenke zumindest teilweise umgreifendes Bügelelement und ein den Unterschenkel oberhalb des oberen Sprunggelenks zumindest teilweise umgreifendes Manschettenelement aufweist, und wobei das Manschettenelement auf der Fußinnenseite und/oder auf der Fußaußenseite mit dem Bügelelement über (jeweils) ein Gelenk verbunden ist zur Übertragung von zumindest Zugkräften zwischen Bügelelement und Manschettenelement, vorgeschlagen, daß das fußaußenseitige Gelenk gegenüber einer Mittelposition zwischen dem äußeren Knöchel und dem inneren Knöchel des oberen Sprunggelenks in Schuh-Längsrichtung nach vorne versetzt angeordnet ist, bzw. daß das fußinnenseitige Gelenk gegenüber der Mittelposition in Schuh-Längsrichtung nach hinten versetzt angeordnet ist. Hierdurch erreicht man bei ausreichender Beweglichkeit einen zuverlässigen Verletzungsschutz. Die erfindungsgemäße Verstärkung läßt sich auch in Verbindung mit einem orthopädischen Strumpf oder dergleichen textilen Stützgeweben zur Sprunggelenkstabilisierung einsetzen.

## Patentansprüche

1. Schuh, insbesondere Sportschuh, umfassend:
A) eine Verstärkung (20), die den Fuß unterhalb der Sprunggelenke teilweise oder vollständig umfaßt und seitlich des Fußes bis oberhalb des oberen Sprunggelenks reicht, wobei die Verstärkung (20) ein den Fuß unterhalb der Sprunggelenke zumindest teilweise umgreifendes Bügelelement (28) und ein den Unterschenkel oberhalb des oberen Sprunggelenks zumindest teilweise umgreifendes Manschettenelement (30) aufweist;
B) Gelenkmittel, bestehend aus einem fußaußenseitigen Gelenk (32) und einem fußinnenseitigen Gelenk (34), die das Manschettenelement (30) auf der Fußaußenseite und auf der Fußinnenseite mit dem Bügelelement (28) verbinden zur Übertragung zumindest von Zugkräften zwischen Bügelelement (28) und Manschettenelement (30);
C) wobei der Schuh eine effektive, durch die Gelenkmittel (32, 34) definierte Bewegungsachse (G) aufweist, die - in Projektion auf eine Horizontalebene - bezüglich einer Querachse (B) des Schuhs derart entgegengesetzt zur Orientierung der die Bewegung der Fußwurzel unter dem oberen Sprunggelenk charakterisierenden Henkeschen Achse orientiert ist, daß ein Verletzungsschutz für den Schuhträger erreicht wird;
D) wobei zur Erzielung der den Verletzungsschutz gebenden Orientierung der Bewegungsachse (G) das fußaußenseitige Gelenk (32) gegenüber einer Mittelposition (M) zwischen dem äußeren Knöchel (24) und dem inneren Knöchel (26) des oberen Sprunggelenks in Schuh-Längsrichtung (A) so weit nach vorne versetzt angeordnet und das fußinnenseitige Gelenk (34) gegenüber der Mittelposition (M) in Schuh-Längsrichtung (A) so weit nach hinten versetzt angeordnet ist, daß die Bewegungsachse (G) zwischen den beiden Gelenken (32, 34) und die Schuh-Querachse (B) in Projektion auf die Horizontalebene einen Winkel (a) von wenigstens 10° einschließen.

2. Schuh, insbesondere Sportschuh, umfassend:
A) eine Verstärkung (20), die den Fuß unterhalb der Sprunggelenke teilweise oder vollständig umfaßt und seitlich des Fußes bis oberhalb des oberen Sprunggelenks reicht, wobei die Verstärkung (20) ein den Fuß unterhalb der Sprunggelenke zumindest teilweise umgreifendes Bügelelement (28) und ein den Unterschenkel oberhalb des oberen Sprunggelenks zumindest teilweise umgreifendes Manschettenelement (30) aufweist;
B) Gelenkmittel, bestehend aus einem fußaußenseitigen Gelenk (32), das das Manschettenelement (30) auf der Fußaußenseite mit dem Bügelelement (28) verbindet zur Übertragung zumindest von Zugkräften zwischen Bügelelement (28) und Manschettenelement (30);
C) wobei der Schuh eine effektive, durch die Gelenkmittel (32) definierte Bewegungsachse (G) aufweist, die - in Projektion auf eine Horizontalebene - bezüglich einer Querachse (B) des Schuhs derart entgegengesetzt zur Orientierung der die Bewegung der Fußwurzel unter dem oberen Sprunggelenk charakterisierenden Henkeschen Achse orientiert ist, daß ein Verletzungsschutz für den Schuhträger erreicht wird;
D) wobei zur Erzielung der den Verletzungsschutz gebenden Orientierung der Bewegungsachse (G) das fußaußenseitige Gelenk (32) gegenüber einer Mittelposition (M) zwischen dem äußeren Knöchel (24) und dem inneren Knöchel (26) des oberen Sprunggelenks in Schuh-Längsrichtung (A) soweit nach vorne versetzt angeordnet ist, daß eine durch das fußaußenseitige Gelenk (32) und einen der Mittelposition entsprechenden Mittelpunkt (M) einer Verbindungsstrecke (H) zwischen dem äußeren Knöchel (24) und dem inneren Knöchel (26) des oberen Sprunggelenks gehende Vertikalebene und die Schuh-Querachse (B) einen Winkel (a) von wenigstens 10° einschließen.

3. Schuh, insbesondere Sportschuh, umfassend:
A) eine Verstärkung (20), die den Fuß unterhalb der Sprunggelenke teilweise oder vollständig umfaßt und seitlich des Fußes bis oberhalb des oberen Sprunggelenks reicht, wobei die Verstärkung (20) ein den Fuß unterhalb der Sprunggelenke zumindest teilweise umgreifendes Bügelelement (28) und ein den Unterschenkel oberhalb des oberen Sprunggelenks zumindest teilweise umgreifendes Manschettenelement (30) aufweist;
B) Gelenkmittel, bestehend aus einem fußinnenseitigen Gelenk (34), das das Manschettenelement (30) auf der Fußinnenseite mit dem Bügelelement (28) verbindet zur Übertragung zumindest von Zugkräften zwischen Bügelelement (28) und Manschettenelement (30);
C) wobei der Schuh eine effektive, durch die G Lenkmittel (34) definierte Bewegungsachse (G) aufw st, die in Projektion auf eine Horizontalebene - bezüglich einer Querachse (B) des Schuhs derart entgegengesetzt zur Orientierung der die Bewegung der Fußwurzel unter dem oberen Sprunggelenk charakterisierenden Henkeschen Achse orientiert ist, daß ein Verletzungsschutz für den Schuhträger erreicht wird;
D) wobei zur Erzielung der den Verletzungsschutz gebenden Orientierung der Bewegungsachse (G) das fußinnenseitige Gelenk (34) gegenüber einer Mittelposition (M) zwischen dem äußeren Knöchel (24) und dem inneren Knöchel (26) des oberen Sprunggelenks in Schuh-Längsrichtung (A) soweit nach hinten versetzt angeordnet ist, daß eine durch das fußinnenseitige Gelenk (34) und einen der Mittelposition entsprechenden Mittelpunkt (M) einer Verbindungsstrecke (H) zwischen dem äußeren Knöchel (24) und dem inneren Knöchel (26) des oberen Sprunggelenks gehende Vertikalebene und die Schuh-Querachse (B) einen Winkel (a) von wenigstens 10° einschließen.

4. Orthopädischer Strumpf, umfassend:
A) eine Verstärkung (120), die den Fuß unterhalb der Sprunggelenke teilweise oder vollständig umfaßt und seitlich des Fußes bis oberhalb des oberen Sprunggelenks reicht, wobei die Verstärkung (120) ein den Fuß unterhalb der Sprunggelenke zumindest teilweise umgreifendes Bügelelement (128) und ein den Unterschenkel oberhalb des oberen Sprunggelenks zumindest teilweise umgreifendes Manschettenelement (30) aufweist;
B) Gelenkmittel, bestehend aus einem fußaußenseitigen Gelenk (132) und einem fußinnenseitigen Gelenk (134), die das Manschettenelement (130) auf der Fußaußenseite und auf der Fußinnenseite mit dem Bügelelement (128) verbinden zur Übertragung zumindest von Zugkräften zwischen Bügelelement (128) und Manschettenelement (130);
C) wobei der Strumpf eine effektive, durch die Gelenkmittel (132, 134) definierte Bewegungsachse aufweist, die - in Projektion auf eine Horizontalebene - bezüglich einer Querachse des Strumpfes derart entgegengesetzt zur Orientierung der die Bewegung der Fußwurzel unter dem oberen Sprunggelenk charakterisierenden Henkeschen Achse orientiert ist, daß ein Verletzungsschutz für den Strumpfträger erreicht wird;
D) wobei zur Erzielung der den Verletzungsschutz gebenden Orientierung der Bewegungsachse das fußaußenseitige Gelenk (132) gegenüber einer Mittelposition zwischen dem äußeren Knöchel (24) und dem inneren Knöchel (26) des oberen Sprunggelenks in Strumpf-Längsrichtung (A') so weit nach vorne versetzt angeordnet und das fußinnenseitige Gelenk (134) gegenüber der Mittelposition in Strumpf-Längsrichtung (A') so weit nach hinten versetzt angeordnet ist, daß eine Verbindungsgerade zwischen den beiden Gelenken (132, 134) und die Strumpf-Querachse in Projektion auf die Horizontalebene einen Winkel (α) von wenigstens 10° einschließen.

5. Schuh oder orthopädischer Strumpf nach einem der vorhergehenden Ansprüche,
dadurch gekennzeichnet, daß
das fußaußenseitige Gelenk (32) gegenüber dem äußeren Knöchel (24) des oberen Sprunggelenks nach unten versetzt angeordnet ist bzw. daß das fußinnenseitige Gelenk (34) gegenüber dem inneren Knöchel (26) des oberen Sprunggelenks nach unten versetzt angeordnet ist.

6. Schuh oder orthopädischer Strumpf nach einem der Ansprüche 1 bis 5,
dadurch gekennzeichnet, daß
der Winkel α 10° bis 30°, vorzugsweise etwa 20°, beträgt.

7. Schuh oder orthopädischer Strumpf nach Anspruch 1 oder 4 oder nach Anspruch 5 oder 6, wenn auf Anspruch 1 oder 4 rückbezogen,
dadurch gekennzeichnet, daß
das fußaußenseitige Gelenk (32) höher als das fußinnenseitige Gelenk (34) angeordnet ist und die Projektion (P) der Verbindungsgeraden (G) zwischen den beiden Gelenken (32, 34) auf eine zur Schuh-Längsrichtung (A) senkrechte, die Schuh-Querachse (B) enthaltende Vertikalebene und die Schuh-Querachse (B) einen Winkel β von 5° bis 15°, vorzugsweise von etwa 10°, einschließen.

8. Schuh oder orthopädischer Strumpf nach einem der vorhergehenden Ansprüche,
dadurch gekennzeichnet, daß
das fußinnenseitige Gelenk (34) bzw. das fußaußenseitige Gelenk (32) etwa auf halber Höhe zwischen der Position des äußeren Knöchels (24) bzw. der Position des inneren Knöchels (26) des oberen Sprunggelenks und der Oberseite der Innensohle angeordnet ist.

9. Schuh nach einem der Ansprüche 1 bis 3, 5 bis 8,
dadurch gekennzeichnet, daß
der Schuh ein einschaliger Schuh ist.

10. Schuh nach einem der Ansprüche 1 bis 3, 5 bis 9,
dadurch gekennzeichnet, daß
der Schuh ein zweischaliger Schuh ist.

11. Schuh nach Anspruch 10,
dadurch gekennzeichnet, daß
das Bügelelement (28), das Manschettenelement (30) und das Gelenk (32; 34) bzw. die Gelenke (32, 34) am Innenschuh (10) des zweischaligen Schuhs angeordnet sind.

12. Schuh nach Anspruch 10,
dadurch gekennzeichnet, daß
das Bügelelement (28), das Manschettenelement (30) und das Gelenk (32; 34) bzw. die Gelenke (32, 34) an der Innenseite des Außenschuhs des zweischaligen Schuhs angeordnet sind.

13. Schuh nach Anspruch 10,
dadurch gekennzeichnet, daß
das Bügelelement (28), das Manschettenelement (30) und das Gelenk (32; 34) bzw. die Gelenke (32, 34) an der Außenseite des Außenschuhs des zweischaligen Schuhs angeordnet sind.

14. Schuh nach der Anspruch 9,
dadurch gekennzeichnet, daß
das Bügelelement (28), das Manschettenelement (30) und das Gelenk (32; 34) bzw. die Gelenke (32, 34) an der Außenseite des Schuhs angeordnet sind.

15. Schuh nach einem der vorhergehenden Ansprüche,
dadurch gekennzeichnet,
daß das Manschettenelement (30) bereichsweise den Schaft (14) des Schuhs bildet.

16. Schuh oder orthopädischer Strumpf nach einem der vorhergehenden Ansprüche,
dadurch gekennzeichnet, daß
das Manschettenelement (30) eine den Unterschenkel umgreifende, vorzugsweise durch ein Verschlußelement (22) schließbare Manschette (18) sowie ein zugsteifes Verbindungsteil (36; 38) zwischen Manschette (18) und dem jeweiligen Gelenk (32; 34), vorzugsweise in Form einer Zunge (36; 38) aufweist.

17. Schuh oder orthopädischer Strumpf nach Anspruch 16,
dadurch gekennzeichnet, daß
die Zunge (36; 38) und das Bügelelement (28) drucksteif ausgebildet sind.

18. Schuh oder orthopädischer Strumpf nach Anspruch 17,
dadurch gekennzeichnet, daß
die aus Leder oder dergleichen flexiblem Material gebildete Zunge (36; 38) durch eine Kunststoffplatte (46) verstärkt ist.

19. Schuh oder orthopädischer Strumpf nach Anspruch 1 oder 4 oder nach einem der Ansprüche 5 bis 18,
wenn auf Anspruch 1 oder 4 rückbezogen,
dadurch gekennzeichnet, daß
das Bügelelement (28) als beide Gelenke (32, 34) verbindender, vorzugsweise gesonderter Bügel ausgebildet ist.

20. Schuh oder orthopädischer Strumpf nach einem der Ansprüche 1 bis 18,
dadurch gekennzeichnet, daß
das Bügelelement (28) mit einer Sohle (12) integriert ist.

21. Schuh oder orthopädischer Strumpf nach einem der Ansprüche 1 bis 18,
dadurch gekennzeichnet, daß
zur Bildung des Bügelelements (28) eine insbesondere nach hinten geschlossene Hinterfuß-Fersenschale seitlich bis zu den Ge'enken hochgezogen ist.

22. Schuh oder orthopädischer Strumpf nach einem der vorhergehenden Ansprüche,
dadurch gekennzeichnet, daß
das Bügelelement (28; 228) mit dem Manschettenelement (30; 230; 230') zur Gelenkbildung über ein Gelenkbolzen-Element (40; 42; 240; 242) verbunden ist, welches in eine Ausnehmung, vorzugsweise ein Loch (250; 252; 254; 256; 254'; 256') im Bügelelement (28; 228) und/ oder im Manschettenelement (30; 230; 230') eingreift.

23. Schuh oder orthopädischer Strumpf nach einem der vorhergehenden Ansprüche,
dadurch gekennzeichnet, daß
das bzw. wenigstens ein Gelenk durch eine Materialabschwächung (332, 334) oder dergleichen gebildet ist.

24. Schuh oder orthopädischer Strumpf nach Anspruch 1 oder 4 oder nach einem der Ansprüche 5 bis 23, wenn auf Anspruch 1 oder 4 rückbezogen,
dadurch gekennzeichnet, daß
wenigstens eines der beiden Gelenke (32, 34; 432) mit im wesentlichen vertikalem Gelenkspiel ausgebildet ist.

25. Schuh oder orthopädischer Strumpf nach Anspruch 24,
dadurch gekennzeichnet, daß
das Bügelelement (28) mit dem Manschettenelement (30) zur Gelenkbildung über ein Gelenkbolzen-Element (40; 42) verbunden ist, welches ein Langloch (44) im Bügelelement (28) und/oder im Manschettenelement (30) durchsetzt.

26. Schuh oder orthopädischer Strumpf nach einem der vorhergehenden Ansprüche,
dadurch gekennzeichnet, daß
das bzw. wenigstens ein Gelenk durch einen Faltenbalgabschnitt (432) oder dergleichen gebildet ist.

27. Schuh oder orthopädischer Strumpf nach Anspruch 26,
dadurch gekennzeichnet, daß
die die Falten (460) des Faltenbalgabschnitts (432) verbindenden Teilabschnitte (462) des Faltenbalgabschnitts (432) im wesentlichen in Schuh-Längsrichtung bzw. Strumpf-Längsrichtung verlaufen und sich nach hinten oder nach vorne verbreitern.

28. Schuh oder orthopädischer Strumpf nach einem der vorhergehenden Ansprüche,
dadurch gekennzeichnet, daß
das Manschettenelement (230; 230') bezüglich des Bügelelements (228) abnehmbar ist.

29. Schuh oder orthopädischer Strumpf nach einem der vorhergehenden Ansprüche,
dadurch gekennzeichnet, daß
das Manschettenelement (230) bezüglich des Bügelelements (228) höhenverstellbar ist.

30. Schuh oder orthopädischer Strumpf nach Anspruch 28 oder 29,
dadurch gekennzeichnet, daß
wenigstens zwei unterschiedliche Manschettenelemente (230, 230'), insbesondere mit unterschiedlichen Abmessungen insbesondere in Vertikalrichtung vorgesehen sind, die wahlweise zur Anpassung des Schuhes bzw. Strumpfes an die Anatomie des Trägers und/oder zur Anpassung des Schuhes bzw. des Strumpfes an den Verwendungszweck mit dem Bügelelement (228) verbindbar sind.

31. Schuh oder orthopädischer Strumpf nach einem der Ansprüche 1 bis 27,
dadurch gekennzeichnet, daß
das Manschettenelement (330) und das Bügelelement (328) einstückig ausgebildet sind.

32. Orthopädischer Strumpf nach Anspruch 4 oder nach einem der Ansprüche 5 bis 8, wenn auf Anspruch 4 rückbezogen,
dadurch gekennzeichnet, daß
das Bügelelement (128) von einem zugfesten Band gebildet ist.

33. Orthopädischer Strumpf nach Anspruch 4 oder 32 oder nach einem der Ansprüche 5 bis 8, wenn auf Anspruch 4 rückbezogen,
dadurch gekennzeichnet, daß
das Manschettenelement (130) von einem den Unterschenkel umgreifenden zugfesten Verschlußband (122) und einem fußinnenseitigen und einem fußaußenseitigen zugfesten Verbindungsteil (136, 138), vorzugsweise in Form einer Zunge (136, 138), zwischen Verschlußband (122) und dem jeweiligen Gelenk (132, 134) gebildet ist.

34. Orthopädischer Strumpf nach Anspruch 33,
dadurch gekennzeichnet, daß
die Zungen (136, 138) von zugfesten Bändern gebildet sind.

## Claims

1. Shoe, in particular sport shoe, with
A) a reinforcement (20), which partially or entirely encloses the foot below the ankle joints and which extends above the upper ankle joint on the sides of the foot, wherein the reinforcement (20) has a stirrup element (28) which at least partially surrounds the foot below the ankle joints and a sleeve element (30) which surrounds the calf above the upper ankle joint at least partially;
B) means of articulation, consisting of an outer side of the foot articulation (32) and an inner side of the foot articulation (34) which connect the sleeve element (30) on the outer side of the foot and on the inner side of the foot with the stirrup element (28), in order to transmit at least tractive forces between the stirrup element (28) and the sleeve element (30);
C) wherein the shoe has an effective axis of movement (G) defined by the means of articulation (32, 34) which, projected on a horizontal plane, with reference to a transverse axis (B) of the shoe, is opposed in orientation in such a way to the orientation of the Henke axis, characterising the movement of the tarsus below the upper ankle joint, that a protection against injury is achieved for the wearer of the shoe;
D) wherein, in order to achieve the orientation of the axis of movement (G) providing protection against injury, the foot outer-side articulation (32) is arranged so as to be offset so far to the front, in relation to a median position (M) between the outer ankle (24) and the inner ankle (26) of the upper ankle joint in the longitudinal direction of the shoe (A), and the inner side of the foot articulation (34) is arranged to be offset so far to the rear in relation to the median position (M) in the longitudinal direction of the shoe, that the axis of movement (G) between both articulations (32, 34) and the transverse axis (B) of the shoe projected onto the horizontal plane, enclose an angle of at least 10°.

2. Shoe, in particular sport shoe, comprising:
A) a reinforcement (20) which surrounds the foot below the ankle joint partly or completely and reaches to above the upper ankle joint to the side of the foot, wherein the reinforcement (20) comprises a stirrup element (28) at least partially surrounding the foot below the ankle joints and a sleeve element (30) at least partially surrounding the calf above the upper ankle joint;
B) means of articulation, consisting of an outer side of the foot articulation (32) which connects the sleeve element (30) to the stirrup element (28) on the outer side of the foot, to transmit at least tractive forces between stirrup element (28) and sleeve element (30);
C) wherein the shoe comprises an effective axis of movement (G) defined by the means of articulation (32), which, projected onto a horizontal plane, with reference to a transverse axis (B) of the shoe is orientated in a manner opposed to the orientation of the Henke axis characterising the movement of the tarsus below the upper ankle joint in such a way that a protection from injury is achieved for the wearer of the shoe;
D) wherein in order to achieve the orientation of the axis of movement (G) providing the protection from injury, the outer side of the foot articulation (32) is arranged so far offset to the front in relation to a median position (M) between the outer ankle (24) and the inner ankle (26) of the upper ankle joint in the longitudinal direction of the shoe (A), that a vertical plane extending through the outer side of the foot articulation (32) and through a centre point (M) corresponding to the median position, of a connecting line (H) between the outer ankle (24) and the inner ankle (26) of the upper ankle joint and the transverse axis of the shoe (B) enclose an angle (α) of at least 10°.

3. Shoe, in particular sport shoe, comprising:
A) a reinforcement (20) which surrounds the foot below the ankle joint partially or completely and extends to the side of the foot to above the upper ankle joint, wherein the reinforcement (20) comprises a stirrup element (28) surrounding the foot below the ankle joint at least partially, and a sleeve element surrounding the calf above the upper ankle joint at least partially;
B) means of articulation, consisting of an inner side of the foot articulation (34) which connects the sleeve element (30) on the inner side of the foot with the stirrup element (28) for the transfer at least of tractive forces between the sleeve element (28) and sleeve element (30);
C) wherein the shoe comprises an effective axis of movement (G) defined by the means of articulation (34), which, projected onto a horizontal plane, with reference to a transverse axis (B) of the shoe, is orientated in a manner opposed to the orientation of the Henke axis characterising the movement of the tarsus below the upper ankle joint in such a way, that a protection from injury is achieved for the wearer of the shoe;
D) wherein to achieve the orientation of the axis of movement (G) providing the protection from injury, the inner side of the foot articulation (34) is arranged offset so far to the rear in relation to a median position (M) between the outer ankle (24) and the inner ankle (26) of the upper ankle joint in the longitudinal direction of the shoe (A), that a vertical plane extending through the inner side of the foot articulation (34) and a central point (M) corresponding to the median position, of a connecting line (H) between the outer ankle (24) and the inner ankle (26) of the upper ankle joint, and the transverse axis (B) of the shoe enclose an angle (α) of at least 10°.

4. Orthopaedic stocking, comprising:
A) a reinforcement (120) which completely or partially encloses the foot below the ankle joints and which extends above the upper ankle joint on the side of the foot, wherein the reinforcement (120) has a stirrup element (128) which surrounds the foot below the ankle joints at least partially and a sleeve element (30) surrounding the calf above the upper ankle joint at least partially;
B) means of articulation, consisting of an outer side of the foot articulation (132) and an inner side of the foot articulation (134) which connect the sleeve element (130) on the outer side of the foot and on the inner side of the foot with the stirrup element (128) for the transfer at least of tractive forces between stirrup element (128) and sleeve element (130);
C) wherein the stocking comprises an effective axis of movement defined by the means of articulation (132, 134), which, projected onto a horizontal plane, with reference to a transverse axis of the stocking, is orientated in a manner opposed to the orientation of the Henke axis characterising the movement of the tarsus below the upper ankle joint in such a way that a protection from injury is achieved for the wearer of the stocking;
D) wherein to achieve the orientation of the axis of movement providing the protection from injury, the foot outer side articulation (132) is arranged so far offset to the front in relation to a median position between the outer ankle (24) and the inner ankle (26) of the upper ankle joint in the longitudinal direction of the stocking (A'), and the foot inner side articulation (134) is arranged offset so far to the rear in relation to the median position in the longitudinal direction of the stocking (A'), that a connecting line between the two articulations (132, 134) and the transverse axis of the stocking projected onto the horizontal plane enclose an angle (α) of at least 10°.

5. Shoe or orthopaedic stocking according to one of the preceding claims, characterised in that the outer side of the foot articulation (32) is arranged offset downwards in relation to the outer ankle (24) of the upper ankle joint, or in that the inner side of the foot articulation (34) is arranged offset downwards in relation to the inner ankle (26) of the upper ankle joint.

6. Shoe or orthopaedic stocking according to one of claims 1 to 5, characterised in that the angle (α) is 10° to 30°, preferably about 20°.

7. Shoe or orthopaedic stocking according to claim 1 or 4, or according to claim 5 or 6 when referred back to claim 1 or 4, characterised in that the outer side of the foot articulation (32) is arranged higher than the inner side of the foot articulation (34) and the projection (P) of the connecting line (G) between the two articulations (32, 34) onto a vertical plane, vertical to the longitudinal direction of the shoe (A) and containing the transverse axis of the shoe (B), and the transverse axis of the shoe (B) enclose an angle (β) of 5° to 15°, preferably of about 10°.

8. Shoe or orthopaedic stocking according to one of the preceding claims, characterised in that the inner side of the foot articulation (34) or the outer side of the foot articulation (32) is arranged at a height about halfway between the position of the outer ankle (24) or the position of the inner ankle (26) of the upper ankle joint and the upper side of the inner sole.

9. Shoe according to one of claims 1 to 3, 5 to 8, characterised in that the shoe is a one-piece shoe.

10. Shoe according to one of claims 1 to 3, 5 to 9, characterised in that the shoe is a two-piece shoe.

11. Shoe according to claim 10, characterised in that the stirrup element (28), the sleeve element (30), and the articulation (32; 34) or articulations (32, 34) are arranged at the inner shoe (10) of the two-piece shoe.

12. Shoe according to claim 10, characterised in that the stirrup element (28), the sleeve element (30) and the articulation (32; 34) or the articulations (32, 34) are arranged at the inner side of the outer shoe of the two-piece shoe.

13. Shoe according to claim 10, characterised in that the stirrup element (28), the sleeve element (30) and the articulation (32; 34) or the articulations (32, 34) are arranged at the outer side of the outer shoe of the two-piece shoe.

14. Shoe according to claim 9, characterised in that the stirrup element (28), the sleeve element (30) and the articulation (32; 34) or the articulations (32, 34) are arranged at the outer side of the shoe.

15. Shoe according to one of the preceding claims, characterised in that the sleeve element (30) forms regions of the body (14) of the shoe.

16. Shoe or orthopaedic stocking according to one of the preceding claims, characterised in that the sleeve element (30) comprises a sleeve (18) surrounding the calf, which can be closed preferably by a closing element (22) and a connecting part (36; 38) which is rigid against traction, between sleeve (18) and the respective articulation (32; 34), preferably in the form of a tongue (36; 38).

17. Shoe or orthopaedic stocking according to claim 16, characterised in that the tongue (36; 38) and the stirrup element (28) are constructed so as to be rigid against pressure.

18. Shoe or orthopaedic stocking according to claim 17, characterised in that the tongue (36; 38) constructed from leather or similar flexible material is reinforced by a plastic plate (46).

19. Shoe or orthopaedic stocking according to claim 1 or 4, or according to one of claims 5 to 18, when referred back to claim 1 or 4, characterised in that the stirrup element (28) is designed as stirrup connecting the two articulations (32, 34) preferably as a separate stirrup.

20. Shoe or orthopaedic stocking according to one of claims 1 to 18, characterised in that the stirrup element (28) is integrated with a sole (12).

21. Shoe or orthopaedic stocking according to one of claims 1 to 18, characterised in that a rear heel shell which is in particular closed at the rear, is raised at the sides up to the articulations in order to form the stirrup element (28).

22. Shoe or orthopaedic stocking according to one of the preceding claims, characterised in that the stirrup element (28; 228) is connected to the sleeve element (30; 230; 230') for forming the articulation via an articulated pin element (40; 42; 240; 242) which engages in a recess, preferably a hole (250; 252; 254; 256; 254'; 256') in the stirrup element (28; 228) and/or in the sleeve element (30; 230; 230').

23. Shoe or orthopaedic stocking according to one of the preceding claims, characterised in that the at least one articulation is formed by a reduction of material (332, 334) or similar.

24. Shoe or orthopaedic stocking according to claim 1 or 4, or according to one of claims 5 to 23, when referred back to claim 1 or 4, characterised in that at least one of the two articulations (32, 34; 432) is constructed with substantially vertical articulation play.

25. Shoe or orthopaedic stocking according to claim 24, characterised in that the stirrup element (28) is connected to the sleeve element (30) for the formation of articulation via a hinge pin element (40; 42), which penetrates a slot (44) in the stirrup element (28) and/or in the sleeve element (30).

26. Shoe or orthopaedic stocking according to one of the preceding claims, characterised in that the or at least one articulation is formed by a bellows portion (432) or similar.

27. Shoe or orthopaedic stocking according to claim 26, characterised in that the partial portions (462) of the bellow portion (432) which connect the folds (460) of the bellows portion (432) extends substantially in the longitudinal direction of the shoe or stocking and widen toward the rear or toward the front.

28. Shoe or orthopaedic stocking according to one of the preceding claims, characterised in that the sleeve element (230; 230') is removable with respect to the stirrup element (228).

29. Shoe or orthopaedic stocking according to one of the preceding claims, characterised in that the sleeve element (230) is vertically adjustable relative to the stirrup element (228).

30. Shoe or orthopaedic stocking according to claim 28 or 29, characterised in that there are at least two different sleeve elements (230, 230') particularly with different dimensions especially in the vertical direction, these sleeve elements being connectable to the stirrup element (228) optionally for adapting the shoe or stocking to the anatomy of the wearer and/or to adapt the shoe or stocking to the intended use.

31. Shoe or orthopaedic stocking according to one of claims 1 to 27, characterised in that the sleeve element (330) and the stirrup element (328) are constructed in one piece.

32. Orthopaedic stocking according to claim 4, or according to claims 5 to 8, when referred back to claim 4, characterised in that stirrup element (128) is formed by a strap which is rigid against traction.

33. Orthopaedic stocking according to claim 4 or 32, or according to one of claims 5 to 8, when referred back to claim 4, characterised in that the sleeve element (130) is formed by a closing strap (122) which engages around the calf and is rigid against traction and by a connecting part (136, 138), preferably in the form of a tongue (136, 138) on the inner side and outer side of the foot between the closing strap (122) and the respective articulation (132, 134), which connecting part (136, 138) is rigid against traction.

34. Orthopaedic stocking according to claim 33, characterised in that the tongues (136, 138) are formed by straps which are rigid against traction.

## Revendications

1. Chaussure, notamment chaussure de sport, comprenant :
A) un renfort (20) qui entoure partiellement ou totalement le pied au-dessous des articulations tibio-tarsiennes et qui s'étend sur le côté du pied jusqu'au-dessus de l'articulation tibio-tarsienne supérieure, le renfort (20) comportant un élément en étrier (28) entourant le pied au-dessous des articulations tibio-tarsiennes, au moins partiellement, ainsi qu'un élément en manchon (30) entourant au moins partiellement la jambe, au-dessus de l'articulation tibio-tarsienne ;
B) des moyens d'articulation constitués d'une articulation (32) côté extérieur du pied et d'une articulation (34) côté intérieur du pied qui relient l'élément en manchon (30), sur le côté extérieur du pied et sur le côté intérieur du pied, à l'élément en étrier (28), pour transmettre au moins des forces de traction entre l'élément en étrier (28) et l'élément en manchon (30) ;
C) la chaussure présentant un axe de mouvement (G) effectif, défini par les moyens d'articulation (32, 34) et qui - en projection sur un plan horizontal - est orienté par rapport à un axe transversal (B) de la chaussure, dans un sens opposé à l'orientation de l'axe de Henke qui caractérise le mouvement du tarse au-dessous de l'articulation tibio-tarsienne supérieure, de manière à obtenir une protection contre des blessures de l'utilisateur de la chaussure ;
D) pour obtenir l'orientation de l'axe de mouvement (G), qui assure la protection contre les blessures, l'articulation (32) côté extérieur du pied 25 étant décalée vers l'avant dans la direction longitudinale (A) de la chaussure, par rapport à une position centrale (M) entre l'os extérieur (24) et l'os intérieur (26) de l'articulation tibio-tarsienne supérieure, et l'articulation (34) côté intérieur du pied étant décalée vers l'arrière dans la direction longitudinale (A) de la chaussure, par rapport à la position centrale (M), suffisamment pour que l'axe de mouvement (G) entre les deux articulations (32, 34) et l'axe transversal (B) de la chaussure forment un angle (α) d'au moins 10° en projection sur le plan horizontal.

2. Chaussure, notamment chaussure de sport, comprenant :
A) un renfort (20) qui entoure partiellement ou totalement le pied au-dessous des articulations tibio-tarsiennes et qui s'étend sur le côté du pied jusqu'au-dessus de l'articulation tibio-tarsienne supérieure, le renfort (20) comportant un élément en étrier (28) entourant le pied au-dessous des articulations tibio-tarsiennes, au moins partiellement, ainsi qu'un élément en manchon (30) entourant au moins partiellement la jambe, au-dessus de l'articulation tibio-tarsienne ;
B) des moyens d'articulation constitués d'une articulation (32) côté extérieur du pied qui relient l'élément en manchon (30), sur le côté extérieur du pied, à l'élément en étrier (28), pour transmettre au moins des forces de traction entre l'élément en étrier (28) et l'élément en manchon (30) ;
C) la chaussure présentant un axe de mouvement (G) effectif, défini par les moyens d'articulation (32) et qui - en projection sur un plan horizontal - est orienté par rapport à un axe transversal (B) de la chaussure, dans un sens opposé à l'orientation de l'axe de Henke qui caractérise le mouvement du tarse au-dessous de l'articulation tibio-tarsienne supérieure, de manière à obtenir une protection contre des blessures de l'utilisateur de la chaussure ;
D) pour obtenir l'orientation de l'axe de mouvement (G), qui assure la protection contre les blessures, l'articulation (32) côté extérieur du pied étant décalée vers l'avant dans la direction longitudinale (A) de la chaussure, par rapport à une position centrale (M) entre l'os extérieur (24) et l'os intérieur (26) de l'articulation tibio-tarsienne supérieure, suffisamment loin pour qu'un plan vertical qui passe par l'articulation (32) côté extérieur du pied et par un point central (M) correspondant à la position centrale d'une distance de liaison (H) entre l'os extérieur (24) et l'os intérieur (26) de l'articulation tibio-tarsienne supérieure, et l'axe transversal (B) de la chaussure, forment un angle (α) d'au moins 10°.

3. Chaussure, notamment chaussure de sport, comprenant :
A) un renfort (20) qui entoure partiellement ou totalement le pied au-dessous des articulations tibio-tarsiennes et qui s'étend sur le côté du pied jusqu'au-dessus de l'articulation tibio-tarsienne supérieure, le renfort (20) comportant un élément en étrier (28) entourant le pied au-dessous des articulations tibio-tarsiennes, au moins partiellement, ainsi qu'un élément en manchon (30) entourant au moins partiellement la jambe, au-dessus de l'articulation tibio-tarsienne ;
B) des moyens d'articulation constitués d'une articulation (34) côté intérieur du pied qui relient l'élément en manchon (30), sur le côté intérieur du pied, à l'élément en étrier (28), pour transmettre au moins des forces de traction entre l'élément en étrier (28) et l'élément en manchon (30) ;
C) la chaussure présentant un axe de mouvement (G) effectif, défini par les moyens d'articulation (34) et qui - en projection sur un plan horizontal - est orienté par rapport à un axe transversal (B) de la chaussure, dans un sens opposé à l'orientation de l'axe de Henke qui caractérise le mouvement du tarse au-dessous de l'articulation tibio-tarsienne supérieure, de manière à obtenir une protection contre des blessures de l'utilisateur de la chaussure ;
D) pour obtenir l'orientation de l'axe de mouvement (G), qui assure la protection contre les blessures, l'articulation (34) côté intérieur du pied étant décalée vers l'arrière par rapport à une position centrale (M) entre l'os extérieur (24) et l'os intérieur (26) de l'articulation tibio-tarsienne supérieure, dans la direction longitudinale (A) de la chaussure, suffisamment loin pour qu'un plan vertical qui passe par l'articulation (34) côté intérieur du pied et un point central (M), correspondant à la position centrale d'un tronçon de liaison (H) entre l'os extérieur (24) et l'os intérieur (26) de l'articulation tibio-tarsienne supérieure, et l'axe transversal (B) de la chaussure, forment un angle (α) d'au moins 10°.

4. Chausson orthopédique, comprenant :
A) un renfort (120) qui entoure partiellement ou totalement le pied au-dessous des articulations tibio-tarsiennes et qui s'étend sur le côté du pied jusqu'au-dessus de l'articulation tibio-tarsienne supérieure, le renfort (120) comportant un élément en étrier (128) entourant le pied au-dessous des articulations tibio-tarsiennes, au moins partiellement, ainsi qu'un élément en manchon (130) entourant au moins partiellement la jambe, au-dessus de l'articulation tibio-tarsienne ;
B) des moyens d'articulation constitués d'une articulation (132) côté extérieur du pied et d'une articulation (134) côté intérieur du pied qui relient l'élément en manchon (130), sur le côté extérieur du pied et sur le côté intérieur du pied, à l'élément en étrier (128), pour transmettre au moins des forces de traction entre l'élément en étrier (128) et l'élément en manchon (130);
C) le chausson présentant un axe de mouvement effectif, défini par les moyens d'articulation (132, 134) et qui - en projection sur un plan horizontal - est orienté par rapport à un axe transversal du chausson, dans un sens opposé à l'orientation de l'axe de Henke qui caractérise le mouvement du tarse au-dessous de l'articulation tibio-tarsienne supérieure, de manière à obtenir une protection contre des blessures de l'utilisateur du chausson ;
D) pour obtenir l'orientation de l'axe de mouvement, qui assure la protection contre les blessures, l'articulation (132) côté extérieur du pied étant décalée vers l'avant dans la direction longitudinale (A') du chausson, par rapport à une position centrale entre l'os extérieur (24) et l'os intérieur (26) de l'articulation tibio-tarsienne supérieure, et l'articulation (134) côté intérieur du pied étant décalée vers l'arrière dans la direction longitudinale (A') du chausson, par rapport à la position centrale, suffisamment loin pour qu'une ligne droite de liaison entre les deux articulations (132, 134) et l'axe transversal du chausson forment un angle (α) d'au moins 10° en projection sur le plan horizontal.

5. Chaussure ou chausson orthopédique selon l'une des revendications précédentes, caractérisé en ce que l'articulation (32) côté extérieur du pied est décalée vers le bas par rapport à l'os extérieur (24) de l'articulation tibio-tarsienne supérieure ou en ce que l'articulation (34) côté intérieur du pied est décalée vers le bas par rapport à l'os intérieur (26) de l'articulation tibio-tarsienne supérieure.

6. Chaussure ou chausson orthopédique selon l'une des revendications 1 à 5, caractérisé en ce que l'angle α est compris entre 10° et 30°, de préférence environ égal à 20°.

7. Chaussure ou chausson orthopédique selon la revendication 1 ou 4 ou selon la revendication 5 ou 6, lorsque celle-ci est dépendante de la revendication 1 ou 4, caractérisé en ce que l'articulation (32) côté extérieur du pied est disposée plus haut que l'articulation (34) côté intérieur du pied et en ce que la projection (P) de la ligne droite de liaison (G) entre les deux articulations (32, 34) sur un plan vertical perpendiculaire à la direction longitudinale (A) de la chaussure et contenant l'axe transversal (B) de la chaussure, et l'axe transversal (B) de la chaussure, forment un angle β de 5° à 15°, de préférence d'environ 10°.

8. Chaussure ou chausson orthopédique selon l'une des revendications précédentes, caractérisé en ce que l'articulation (34) côté intérieur du pied, ou l'articulation (32) côté extérieur du pied est disposée sensiblement à mi-hauteur entre la position de l'os extérieur (24), respectivement la position de l'os intérieur (26), de l'articulation tibio-tarsienne supérieure et de la face supérieure de la semelle intérieure.

9. Chaussure selon l'une des revendications 1 à 3, 5 à 8, caractérisée en ce la chaussure est une chaussure à coque unique.

10. Chaussure selon l'une des revendications 1 à 3, 5 à 9, caractérisée en ce que la chaussure est une chaussure à double coque.

11. Chaussure selon la revendication 10, caractérisée en ce que l'élément en étrier (28), l'élément en manchon (30) et l'articulation (32 ; 34), respectivement les articulations (32, 34), sont disposés sur le chausson intérieur (10) de la chaussure à double coque.

12. Chaussure selon la revendication 10, caractérisée en ce que l'élément en étrier (28), l'élément en manchon (30) et l'articulation (32 ; 34), respectivement les articulations (32, 34), sont disposés sur la face intérieure de la coque extérieure de la chaussure à double coque.

13. Chaussure selon la revendication 10, caractérisée en ce que l'élément en étrier (28), l'élément en manchon (30) et l'articulation (32 ; 34), respectivement les articulations (32, 34), sont disposés sur la face extérieure de la coque extérieure de la chaussure à double coque.

14. Chaussure selon la revendication 9, caractérisée en ce que l'élément en étrier (28), l'élément en manchon (30) et l'articulation (32 ; 34), respectivement les articulations (32, 34), sont disposés sur la face extérieure de la chaussure.

15. Chaussure selon l'une des revendications précédentes, caractérisée en ce que l'élément en manchon (30) constitue par endroit l'empeigne (14) de la chaussure.

16. Chaussure ou chausson orthopédique selon l'une des revendications précédentes, caractérisé en ce que l'élément en manchon (30) comporte un manchon (18) qui entoure la jambe et qui peut être fermé de préférence par un élément de fermeture (22), ainsi qu'un élément de liaison (36 ; 38) rigide en traction, entre le manchon (18) et l'articulation (32 ; 34) correspondante, de préférence sous la forme d'une languette (36 ; 38).

17. Chaussure ou chausson orthopédique selon la revendication 16, caractérisé en ce que la languette (36 ; 38) et l'élément en étrier (28) sont rigides en compression.

18. Chaussure ou chausson orthopédique selon la revendication 17, caractérisé en ce que la languette (36 ; 38), en cuir ou en un matériau flexible similaire, est renforcée par une plaque en matière plastique (46).

19. Chaussure ou chausson orthopédique selon la revendication 1 ou 4 ou selon l'une des revendications 5 à 18, lorsque celle-ci est dépendante de la revendication 1 ou 4, caractérisé en ce que l'élément en étrier (28) est réalisé sous la forme d'un étrier, de préférence séparé, reliant les deux articulations (32, 34).

20. Chaussure ou chausson orthopédique selon l'une des revendications 1 à 18, caractérisé en ce que l'élément en étrier (28) est intégré dans une semelle (12).

21. Chaussure ou chausson orthopédique selon l'une des revendications 1 à 18, caractérisé en ce que, pour former l'élément en étrier (28), une coque talon-arrière du pied, fermée notamment vers l'arrière, remonte sur les côtés jusqu'aux articulations.

22. Chaussure ou chausson orthopédique selon l'une des revendications précédentes, caractérisé en ce que l'élément en étrier (28 ; 228) est relié à l'élément en manchon (30 ; 230 ; 230') de manière à former une articulation, par l'intermédiaire d'un élément à axe d'articulation (40 ; 42 ; 240 ; 242), qui s'engage dans un évidement, de préférence un trou (250 ; 252 ; 254 ; 256 ; 254' ; 256') ménagé dans l'élément en étrier (28 ; 228) et/ou dans l'élément en manchon (30 ; 230 ; 230').

23. Chaussure ou chausson orthopédique selon l'une des revendications précédentes, caractérisé en ce que l'articulation ou au moins une articulation est constituée par un affaiblissement de la matière (332, 334) ou similaire.

24. Chaussure ou chausson orthopédique selon la revendication 1 ou 4 ou selon l'une des revendications 5 à 23, lorsque celle-ci est dépendante de la revendication 1 ou 4, caractérisé en ce qu'au moins l'une des deux articulations (32, 34 ; 432) présente un jeu d'articulation sensiblement vertical.

25. Chaussure ou chausson orthopédique selon la revendication 24, caractérisé ce que l'élément en étrier (28) est relié à l'élément en manchon (30), pour former une articulation, par l'intermédiaire d'un élément à axe d'articulation (40 ; 42), qui traverse un trou oblong (44) ménagé dans l'élément en étrier (28) et/ou dans l'élément en manchon (30).

26. Chaussure ou chausson orthopédique selon l'une des revendications précédentes, caractérisé en ce que l'articulation ou au moins une articulation est constituée par un segment de soufflet (432) ou similaire.

27. Chaussure ou chausson orthopédique selon la revendication 26, caractérisé en ce que les segments partiels (462) du segment de soufflet (432), qui relient les plis (460) du segment de soufflet (432), s'étendent sensiblement dans la direction longitudinale de la chaussure, respectivement du chausson, et s'élargissent vers l'arrière ou vers l'avant.

28. Chaussure ou chausson orthopédique selon l'une des revendications précédentes, caractérisé en ce que l'élément en manchon (230 ; 230') peut être enlevé par rapport à l'élément en étrier (228).

29. Chaussure ou chausson orthopédique selon l'une des revendications précédentes, caractérisé en ce que l'élément en manchon (230) est réglable en hauteur par rapport à l'élément en étrier (228).

30. Chaussure ou chausson orthopédique selon la revendication 28 ou 29, caractérisé en ce que sont prévus au moins deux éléments en manchon (230, 230') différents, notamment de dimensions différentes en particulier dans la direction verticale, qui peuvent être reliés au choix à l'élément en étrier (228), pour adapter la chaussure ou le chausson à l'anatomie de l'utilisateur et/ou pour adapter la chaussure ou le chausson à l'utilisation envisagée.

31. Chaussure ou chausson orthopédique selon l'une des revendications 1 à 27, caractérisé en ce que l'élément en manchon (330) et l'élément en étrier (328) sont réalisés d'un seul tenant.

32. Chausson orthopédique selon la revendication 4 ou selon l'une des revendications 5 à 8, lorsque celle-ci est dépendante de la revendication 4, caractérisé en ce que l'élément en étrier (128) est constitué par une bande résistante à la traction.

33. Chausson orthopédique selon la revendication 4 ou 32, ou selon l'une des revendications 5 à 8, lorsque celle-ci est dépendante de la revendication 4, caractérisé en ce que l'élément en manchon (130) est constitué par une bande de fermeture (122) résistant à la traction, entourant la jambe et par un élément de liaison (136, 138), résistant à la traction, côté intérieur du pied et côté extérieur du pied, de préférence sous la forme d'une languette (136, 138), entre la bande de fermeture (122) et l'articulation (132, 134) correspondante.

34. Chausson orthopédique selon la revendication 33, caractérisé en ce que les languettes (136, 138) sont constituées par des bandes résistant à la traction.
